# EUROPEAN PATENT APPLICATION

(11) **EP 4 678 090 A1**
(43) Date of publication of application: **14.01.2026**
(21) Application number: 24187761.2
(22) Date of filing: 10.07.2024
(51) Int. Cl.: A61B 5/00, G01L 1/16, G01N 21/17

(54) **A SENSOR DEVICE, A PHOTO-ACOUSTIC IMAGING DEVICE, AND A METHOD FOR READ-OUT OF A DETECTED MEASURAND**

(71) Applicant: Imec VZW, 3001 Leuven (BE); Katholieke Universiteit Leuven KU Leuven Research & Development, 3000 Leuven (BE)
(72) Inventor: LAMBERTI, Fabrice-Roland, 00138 Rome (IT); BROUCKAERT, Joost, 8500 Kortrijk (BE); PIETERS, Cedric, 2880 Bornem (BE); JANSEN, Roelof, 3001 Heverlee (BE); KEULEMANS, Grim, 1030 Schaarbeek (BE); ROTTENBERG, Xavier, 3010 Kessel-Lo (BE); KJELLMAN, Jon, 2800 Kongens Lyngby (DK)
(74) Representative: AWA Sweden AB

(57) **Abstract**

A sensor device (100) comprises: a plurality of sensors (110; 210; 310) configured to be affected by a measurand; a waveguide (120; 220; 320) associated with a group of sensors and configured to receive an optical interrogation signal comprising at least one interrogation wavelength; a plurality of control elements (130; 230; 330), each being individually controllable and configured to control a resonance wavelength of the sensor (110; 210; 310) associated with the control element (130; 230; 330); the sensor device (100) being configured to, for read-out of measurement from a selected sensor (110; 210; 310), tune its resonance wavelength to be aligned with a selected interrogation wavelength; the group of sensors being configured to be controlled for arranging, at a single read-out time point, the selected sensor (110; 210; 310) uniquely aligned with the selected interrogation wavelength for reading out the measurand measured by the selected sensor (110; 210; 310).

## Description

### Technical field

The present description relates to a sensor device comprising a plurality of sensors, each comprising an optical resonance element. The optical resonance element is configured to be affected by a measurand for detecting the measurand. In particular, the present description relates to a photo-acoustic imaging device comprising the sensor device. The present description also relates to a method for read-out of the detected measurand.

### Background

Optical resonance elements are used in various sensor applications. The optical resonance elements define an optical resonance at a wavelength. The optical resonance may be modified by a measurand that is to be measured such that the measurand may be determined based on an effect on optical resonance caused by the measurand.

The optical resonance elements may for instance be used in photo-acoustic imaging, wherein a measurand in form of an acoustic wave may be detected based on the acoustic wave affecting optical resonance of an optical resonance element. The acoustic wave may for instance provide a response from living tissue being subject to an optical pulse which generates the acoustic wave in the tissue. In order for an image to be formed based on detection of the acoustic wave, a large number of optical resonance elements, each associated with a respective sensor, may be needed.

In view of using a plurality of sensors having optical resonance elements, there is a need for an efficient manner of reading signals from the plurality of sensors. In particular, there is a need to allow simultaneous reading of signals from the plurality of sensors while using a simple and scalable scheme for reading the signals.

### Summary

An objective of the present description is to provide control of read-out of optical signals carrying information of detection of a measurand by a plurality of sensors.

This and other objectives are at least partly met by the invention as defined in the independent claims. Preferred embodiments are set out in the dependent claims.

According to a first aspect, there is provided a sensor device comprising: a plurality of sensors, wherein each sensor comprises an optical resonance element, wherein an optical characteristic of the optical resonance element is configured to be affected by a measurand to be measured by the sensor; a waveguide associated with a group of sensors among the plurality of sensors, wherein the waveguide is configured to receive an optical interrogation signal comprising at least one interrogation wavelength; a plurality of control elements, wherein each sensor in the plurality of sensors is associated with a unique, individual control element of the plurality of control elements, wherein each control element is individually controllable and configured to control a resonance wavelength of optical resonance of the sensor associated with the control element for tuning the resonance wavelength of the sensor; wherein the sensor device is configured for the resonance wavelength of each of the sensors in the group of sensors, before read-out of measurements from the group of sensors, being in at least one wavelength range, wherein the at least one interrogation wavelength is outside the at least one wavelength range; wherein the sensor device is configured to, for read-out of measurement from a selected sensor among the group of sensors, tune the resonance wavelength of the selected sensor by controlling the control element to which the selected sensor is associated for aligning the resonance wavelength with a selected interrogation wavelength of the at least one interrogation wavelength; wherein the group of sensors are configured to be controlled for arranging, at a single read-out time point, the selected sensor to be uniquely aligned with the selected interrogation wavelength for reading out the measurand measured by the selected sensor using the selected interrogation wavelength.

The sensor device is configured to provide detection of the measurand by sensors that each comprise an optical resonance element. This implies that information of the detected measurand may be carried in a resonance wavelength of optical resonance of each sensor. Thanks to the sensor device according to the first aspect, read-out of the information carried in resonance wavelengths of a plurality of sensors is provided in a systematic manner, enabling fast read-out of the measurand from the plurality of sensors while ensuring that read out information can be unambiguously tied to a particular sensor in the plurality of sensors.

The sensor device enables read-out of information from the plurality of sensors in such manner that the sensor device may be easily scaled, i.e., provided with a huge number of sensors, while the read out of information from the sensor device still being provided in a fast manner and without requiring extensive hardware (such as numerous light sources providing multiple interrogation wavelengths and/or numerous detectors for detecting optical interrogation signals).

Thanks to the sensor device, each sensor may be set, when read-out is not to be performed for the sensor, such that the resonance wavelength is in the at least one wavelength range. Further, the at least one interrogation wavelength of the optical interrogation signal is not within the wavelength range. This implies that, sensors that are not to be read out will not affect the optical interrogation signal. Hence, the sensors may be controlled such that a point in time when the sensor may affect the optical interrogation signal may be controlled. Thus, the sensor device allows for dynamic selection of which sensors that will affect the optical interrogation signal at a particular read-out time point.

This also implies that the sensors within the group of sensors need not be manufactured with static resonance wavelengths that need to differ from each other. This ensures that the sensor device is not limited to a few sensors being arranged in a group in order to achieve unique, differentiable resonance wavelengths during read-out of information from the sensors.

The sensor device may thus enable a waveguide to be associated with a group of sensors that includes a large number of sensors. The sensors within the group may then be controlled in a time-multiplexed manner for read-out of all of the sensors in the group. Thus, even though the measurand may affect a non-selected sensor during a read-out time point, the non-selected sensor will not have a resonance wavelength interfering with the interrogation wavelength(s) such that the non-selected sensor will not affect the read-out of selected sensors in the group.

Further, all of the sensors may be manufactured with identical optical resonance elements, or with a few different settings of the optical resonance elements. The sensors need not be differentiated from each other during read-out by the optical resonance elements being differently manufactured. Rather, the sensors can be differentiated from each other based on the control of the resonance wavelengths provided by the plurality of control elements. Hence, since the sensors may have identical settings of the optical resonance elements or may have only a few different settings of the optical resonance elements, manufacturing of the sensor device is greatly simplified. The manufacturing of the sensor device may thus provide multiple sensors with common characteristics, such that parallel manufacturing of the multiple sensors is facilitated. In addition, requirements of tolerances in manufacturing of sensors may be relatively lenient since the sensors may be controlled to be aligned with the resonance wavelength during read-out. Thus, the dynamic control of the resonance wavelength may ensure that the resonance wavelength of the sensor is properly aligned to the interrogation wavelength even if the sensor is not perfectly manufactured to provide characteristics corresponding with nominal characteristics.

As used herein, the term *"measurand"* should be construed as any quantity, object, or property intended to be measured. Given as a non-limiting example, the measurand may be a temperature or a mass or another quantity. Given as another non-limiting example, the measurand may be electro-magnetic radiation or an acoustic wave incident on the optical resonance element. The measurand may be representative of an object interacting with or emitting the electro-magnetic radiation or the acoustic wave. Given as yet another non-limiting example, the measurand may be an object that interacts with the optical resonance element.

The optical resonance element may be affected by the measurand in a manner that may be read out using the optical interrogation signal, such that a measurement of the measurand is acquired by the sensor.

The optical resonance element may be affected by the measurand such that any optical characteristic is affected. For instance, the optical resonance is affected, e.g., the measurand may affect the optical resonance element such that a resonance wavelength may be slightly changed. However, the optical resonance element may alternatively or additionally be affected by the measurand such that a transmission, reflection, absorption, or scattering by the optical resonance element may be directly affected.

The optical resonance element defines optical resonances. The optical resonance element may be designed in many different ways for defining the optical resonances. For instance, the optical resonance element may comprise a loop, such as a ring, disk, or sphere, for allowing an optical signal to travel around the loop. At a resonance wavelength, constructive interference occurs for optical signals making different number of round-trips around the loop. Alternatively, the optical resonance element may comprise a Fabry-Pérot resonator, defining an optical resonator between two reflective elements. As yet another alternative, the optical resonance element may comprise a photonic crystal structure.

According to an embodiment, the optical resonance element is a waveguide resonator. Thus, the optical resonance element may provide a well-defined optical path in a waveguide.

It should be realized that each optical resonance element has a set of resonance wavelengths. When a sensor is selected for read-out of a measurement, one of the resonance wavelengths may be tuned such that the resonance wavelength is aligned with the selected interrogation wavelength.

The optical interrogation signal may be considered as having at least one signal component, wherein each signal component has an interrogation wavelength. The optical interrogation signal may be further configured to interact with the optical resonance element. Interaction of the optical interrogation signal with the optical resonance element may be limited to the signal component having an interrogation wavelength within a bandwidth of an optical mode of the optical resonance element. Hence, only the selected interrogation wavelength of the optical interrogation signal with which the resonance wavelength of the optical resonance element of a sensor is aligned may interact with the sensor.

For instance, the optical interrogation signal may be coupled into the optical resonance element in dependence of the interrogation wavelength. Hence, transmission of the signal component by the optical resonance element may depend on how well the interrogation wavelength of the signal component corresponds to the resonance wavelength of the sensor. Signal components having interrogation wavelengths that are not related to the resonance wavelength of a sensor, i.e., interrogation wavelengths that are not spectrally located in the optical mode of the sensor, will not be affected by a measurand affecting the optical resonance element. Such signal components may possibly be affected by being attenuated by the optical resonance element, but the signal components will not be affected by the measurand.

According to an embodiment, the signal component with the selected interrogation wavelength may be coupled into the optical resonance element of the selected sensor such that intensity of the optical interrogation signal is reduced at the signal component with the selected interrogation wavelength. However, it should be realized that, in other embodiments, the optical resonance element may be configured to provide a maximum transmission at the resonance wavelength such that intensity of the optical interrogation signal transmitted by the optical resonance element is high if the selected interrogation wavelength of the signal component matches well with the resonance wavelength.

Each sensor may be configured to sense the measurand by the optical resonance wavelength of the optical resonance element) being changed in dependence of the measurand. The resonance wavelength of the optical resonance element may thus be slightly changed or modulated by the measurand. This implies that interaction of a signal component having the selected interrogation wavelength with the optical resonance element, such as coupling of the signal component of the optical interrogation signal into the optical resonance element, may be altered by the measurand. In this manner, transmission, or alternatively reflection, absorption, or scattering, of the signal component of the optical interrogation signal by the optical resonance element, may be altered by the measurand. This implies that the optical intensity of the signal component of the optical interrogation signal with which the optical resonance element is aligned will be modulated by the measurand.

The optical resonance wavelength of the optical resonance element may define one characteristic of an optical mode. The signal component of the optical interrogation signal may be coupled to the optical mode of the optical resonance element even if the selected interrogation wavelength of the signal component does not exactly match the optical resonance wavelength. Thus, even if the optical resonance wavelength is modulated by the measurand, the selected interrogation wavelength of the signal component of the optical interrogation signal with which the optical resonance element is aligned may still be coupled to the optical mode. However, the interaction between the signal component and the optical resonance element may be affected by the modulation of the resonance wavelength caused by the measurand, such that transmission, reflection, absorption, or scattering, of the optical interrogation signal by the optical resonance element may be affected. Also, or alternatively, the interaction between the signal component with the selected interrogation wavelength and the optical resonance element may be affected by the measurand, such that transmission, reflection, absorption, or scattering of the optical interrogation signal by the optical resonance element may be directly affected by the measurand.

The signal component of the optical interrogation signal may be coupled into the optical resonance element, which may be used for causing transmission, reflection, absorption, or scattering of the signal component. However, the signal component may interact with the optical resonance element in alternative manners, such as to cause a phase change of the signal component.

The optical resonance element may be configured in many different manners such that the measurand alters the resonance wavelength or directly alters transmission, reflection, absorption, or scattering of the signal component with the selected interrogation wavelength of the optical interrogation signal by the optical resonance element.

For instance, the optical resonance element may comprise an optical waveguide forming a loop, which is arranged on a membrane. Alternatively, the optical resonance element is placed in vicinity of the membrane so that it is optically and/or mechanically coupled to the membrane. Thus, a measurand may affect the membrane so as to alter the shape of the membrane such that a length of the loop of the optical waveguide is modulated so as to modulate the optical resonance wavelength. Alternatively, or additionally, an optical property, such as the refractive index of the optical resonance element or of a material surrounding the optical resonance element may be modulated so as to modulate the optical resonance wavelength. An optical resonator coupled to a mechanical system defines an opto-mechanical system.

According to an alternative embodiment, the measurand may affect coupling of the optical interrogation signal into an optical waveguide of the optical resonance element or the measurand may affect loss of the optical interrogation signal in the optical waveguide. Thus, the measurand may alter transmission of the signal component with the selected interrogation wavelength of the optical interrogation signal by the optical resonance element.

The sensor may be configured for the optical resonance element being directly affected by the measurand. For instance, the measurand may be configured to directly affect the resonance wavelength of the optical resonance element. This may for instance be provided by the measurand being an optical signal or a substance affecting the optical resonance element.

However, the sensor may alternatively be configured for the optical resonance element being indirectly affected by the measurand. For instance, the optical resonance element may be arranged on a membrane which may be deflected in response to the measurand, whereby a deflection of the membrane may affect the resonance wavelength of the optical resonance element. This may for instance be provided by the measurand being an acoustical signal incident on the membrane or a mass landing on the membrane affecting the optical resonance element arranged on the membrane.

Thus, it should be realized that the read-out of measurements of measurands using selecting of sensors by controlling the resonance wavelength of the selected sensor(s) may be used for various types of sensors.

The sensor device comprises a plurality of sensors. It should be realized that the sensors may be arranged in a regular manner forming an array of sensors. The plurality of sensors may be arranged in a one-dimensional array being arranged along a line or in a two-dimensional array being arranged in rows and columns. However, it should be realized that the sensors need not necessarily be arranged with a particular spatial relation between the sensors.

It should be realized that the sensor device may comprise a large number of sensors. Since the sensor device allows for read-out of measurements in a systematic manner, the sensor device is particularly suited for use with a large number of sensors. However, it should be realized that the sensor device need not necessarily include a large number of sensors and the use of the systematic manner of selecting sensors for read-out is not limited to any particular minimum number of sensors.

It should be realized that the sensor device may comprise a plurality of waveguides, such that each waveguide is associated with a distinct group of sensors. Thus, the sensors may be divided into a number of different groups. The plurality of waveguides may be configured to receive optical interrogation signals simultaneously for providing simultaneous read-out of different groups of sensors. This may facilitate fast read-out of measurements from the plurality of sensors.

The group of sensors with which the waveguide is associated may correspond to the sensors of a row or a column of the array. However, it should be realized that the waveguide may be arranged in different manners to be associated with the group of sensors. For instance, the waveguide may be associated with a group of sensors forming part of a row or part of a column, or the waveguide may be associated with a group of sensors forming a sub-array across more than one row and more than one column of the array. Also, the group of sensors need not necessarily be arranged in a regular manner and that the sensors in a group need not necessarily be arranged with a particular spatial relation between the sensors.

However, the sensor device may alternatively be configured to provide optical interrogation signals sequentially to different waveguides associated with different groups of sensors. This implies that a single light source or single set of light sources may be provided for providing the optical interrogation signals to the waveguides. In addition, a single detector or a single set of detectors may be provided for detecting the optical interrogation signals carrying information of read-out of measurements. Thus, the sensor device may be associated with a limited hardware for generating and detecting the optical interrogation signals, while allowing read-out of measurements from a large number of sensors.

The waveguide may be any structure providing guiding of an optical signal. The waveguide may be configured to confine light in a cross-section perpendicular to an extension of the waveguide. The waveguide may thus be configured restrict light to follow a path defined by the extension of the waveguide, whereby transmission of light with low loss may be provided. The waveguide may for instance guide light by light being reflected on inner walls of the waveguide by total internal reflection or by a reflective coating being provided on the walls of the waveguide. The waveguide is configured to propagate the optical interrogation signal along the path of the waveguide.

The waveguide may be configured to extend in a path passing all sensors in the group of sensors. The waveguide may be configured to pass at least in vicinity of the optical resonance elements of the sensors in the group of sensors. The optical interrogation signal may thus be coupled into the optical resonance element and this coupling of the optical interrogation signal into the optical resonance element may be used for information of measurement to be carried by the optical interrogation signal.

The optical interrogation signal may comprise a plurality of interrogation wavelengths. The optical interrogation signal may thus comprise a plurality of signal components, wherein each signal components has a unique interrogation wavelength. The optical interrogation signal may thus carry information of measurements from a plurality of sensors. Each signal component of the optical interrogation signal may carry information from measurements from a single sensor. Thanks to the signal components having different interrogation wavelengths, it is possible to differentiate between the read-out measurements from different sensors.

However, it should be realized that the optical interrogation signal may only comprise a single interrogation wavelength. In such case, the optical interrogation signal may be configured to only carry information from read out measurements from a single sensor at a time.

Each sensor may be configured to modulate the optical intensity at the selected interrogation wavelength. The sensor may modulate the optical intensity by the sensor being affected by the measurand. In addition or alternatively to modulating the optical intensity, the sensor may further be configured to modulate a phase of the signal component with the selected interrogation wavelength.

The optical interrogation signal may be formed by laser light comprising signal components at one or more distinct interrogation wavelengths.

However, it should be realized that the optical interrogation signal may in some embodiments not necessarily be formed by laser light, but rather non-coherent light may be used. This may be used for instance with optical sensors, wherein a high sensitivity of the optical sensor is not needed.

The control elements may be any kind of elements that are able to change the resonance wavelength of the optical resonance element of a sensor in a controlled manner. Thus, the resonance wavelength of the optical resonance element of a sensor may be accurately tuned to a desired value using the control element associated with the sensor.

The control element may be configured to affect a characteristic of the optical resonance element so as to cause a change of the optical resonance wavelength. For instance, the control element may be configured to affect a temperature of the optical resonance element which may affect the refractive index of the optical resonance element and may hence in turn affect the resonance wavelength.

However, the control element may be configured to affect the sensor in other manners for controlling the resonance wavelength. The control element may for instance be configured to utilize an electro-optical effect for controlling the resonance wavelength, e.g., by applying an electric field across the optical resonance element, or to utilize a mechano-optical effect for controlling the resonance wavelength, e.g., by physically changing a path length of the optical resonance element.

Each control element may be used for tuning the resonance wavelength of the sensor to the desired value. The optical resonance element may have a natural resonance wavelength when not affected by the control element, for instance, by the control element not being active. The sensor may be configured such that the natural resonance wavelength is in the at least one wavelength range. Thus, the control element need not be active before read-out of measurements from the group of sensors, since the interrogation wavelength is outside the at least one wavelength range such that the sensor will not affect the optical interrogation signal. Hence, only selected sensors need to be controlled by the respective control element for read-out of measurements from the selected sensors.

However, it should be realized that each control element may also be used for tuning the resonance wavelength of the sensor associated with the control element such that the resonance wavelength is brought into the at least one wavelength range. Thus, the control elements may provide different tuning levels for bringing the resonance wavelength into the at least one wavelength range and for aligning the resonance wavelength with the selected interrogation wavelength.

When a sensor is selected for read-out of measurement, non-selected sensors may be set such that resonance wavelengths of the non-selected sensors are in the at least one wavelength range and hence do not coincide with interrogation wavelength(s). The non-selected sensors may be set in the at least one wavelength range by the control elements of the non-selected sensors being inactive (natural resonance wavelength is within the at least one wavelength range) or by the control elements controlling the resonance wavelengths of the non-selected sensors to be within the at least one wavelength range.

The control element tuning the resonance wavelength for aligning the resonance wavelength with a selected interrogation wavelength implies that the selected interrogating wavelength falls within a resonance curve associated with the resonance wavelength. It should be understood that the resonance wavelength is associated with a resonance curve, such that the optical resonance element may interact with the selected interrogation wavelength even if the peak wavelength of the resonance curve does not exactly match the selected interrogation wavelength. Thus, aligning the resonance wavelength with the selected interrogation wavelength does not imply that the peak resonance wavelength is exactly matched to the selected interrogation wavelength. Rather, an effect of the measurand on the optical resonance element may even be easier to read out if the resonance wavelength is tuned such that the selected interrogation wavelength is arranged at a flank of the resonance curve.

The at least one wavelength range is selected to not coincide with the at least one interrogation wavelength. Each of the at least one wavelength range may be referred to herein as a *"storage window",* since the sensors are arranged (stored) with the resonance wavelengths in the storage window when sensors are non-selected and information is not read-out from the sensors. The sensor device may be configured to provide suitably narrow storage windows so as to provide sufficient spectral bandwidth for the interrogation wavelength(s). However, having very narrow storage windows may require control of the resonance wavelengths by the control elements for non-selected sensors (and hence requiring more power consumption for read-out) or may require very accurate control of manufacturing of the sensor device.

The at least one interrogation wavelength may be very accurately defined. For instance, the optical interrogation signal may be based on laser light, such that each interrogation wavelength may correspond to a laser line, which has a very well-defined wavelength. The optical interrogation signal may comprise a plurality of interrogation wavelengths. This allows read-out of a plurality of sensors simultaneously, by a plurality of selected sensors being aligned with respective selected interrogation wavelengths from the plurality of interrogation wavelengths.

The interrogation wavelengths of the plurality of interrogation wavelengths may need to be spectrally separated such that each interrogation wavelength is exclusively affected by a single selected sensor. Since the optical resonance element may be associated with a resonance curve, the interrogation wavelengths may need to be spectrally separated such that the interrogation wavelength does not fall within the resonance curve of a sensor for which the resonance wavelength is aligned with a neighboring interrogation wavelength. This implies that a signal component with the selected interrogation wavelength in the optical interrogation signal may be exclusively affected by the selected sensor that is aligned to the selected interrogation wavelength.

Each interrogation wavelength may be arranged within a wavelength band that does not coincide with any storage window. A wavelength band in which a single interrogation wavelength is arranged may be referred to as a *"read-out window".* The read-out window may be related to adjacent read-out windows such that when selected sensors are aligned with selected interrogation wavelengths at adjacent read-out windows, the measurements of the selected sensors may be discerned from the optical interrogation signal. Thus, according to an embodiment, a size of a wavelength band in which a single interrogation wavelength is arranged corresponds to a size of a resonance curve of the optical resonance element. However, this may imply that read-out windows may be relatively large and that only a few interrogation wavelengths may be included within the optical interrogation signal. Thus, by having narrower read-out windows, the number of interrogation wavelengths within the optical interrogation signal may be increased allowing measurements from more sensors to be read out simultaneously.

According to another embodiment, a size of a wavelength band in which a single interrogation wavelength is arranged corresponds to a width of a resonance curve of the optical resonance element, wherein a value of the resonance curve is larger than a threshold percentage of a peak value of the resonance curve, wherein the threshold percentage may be for example in a range of 1-10%. This may allow the resonance windows to be narrow but still ensure that a signal component with the selected interrogation wavelength is only marginally affected by a sensor being aligned with an adjacent interrogation wavelength. Thus, crosstalk between different selected sensors may be at least relatively low to allow discerning measurements from different sensors in the optical interrogation signal.

The resonance curve may provide a value of the optical characteristic in dependence of wavelength. The value of the optical characteristic may thus deviate from a baseline value having a peak value corresponding to the peak resonance wavelength. It should be realized that the peak value need not necessarily increase from the baseline value but may rather decrease from the baseline value. Thus, the resonance curve may define a difference between the peak value and the baseline value. The value of the resonance curve being for example at least 10% of the peak value may thus be construed as the value of the resonance curve deviating from the baseline value for example by at least 10% of the deviation by the peak value from the baseline value.

The threshold percentage value can be set by determining the amount of crosstalk between sensors that the measurement can tolerate. The amount of crosstalk can be quantified theoretically or experimentally for example by determining the magnitude of the signal generated in a considered readout window due to the presence of optical modes in the neighboring readout windows. This value can be compared to the magnitude of signal measured when an optical mode is aligned in the considered readout window.

The sensor device is configured such that at a particular point in time when a measurement from a selected sensor is to be read out, the selected sensor within the group of sensors is uniquely aligned with the selected interrogation wavelength, i.e., the selected sensor is the only sensor within the group that is aligned with the selected interrogation wavelength at the particular point in time. This implies that the optical interrogation signal may carry information of the measurement by the selected sensor in the signal component with the selected interrogation wavelength, without the signal component being affected by the measurand measured by any other sensor.

If a plurality of interrogation wavelengths is used, a plurality of sensors in the group may be simultaneously aligned with respective interrogation wavelengths, such that one sensor is aligned with each one of the interrogation wavelengths.

The measurement from the selected sensor may be read out at a read-out time point. This implies that the selected sensor may be sampled at the read-out time point. However, the selected sensor may be selected for read-out during a time duration allowing a time trace of the measurand to be acquired. Thus, selection of the sensor for read-out may be maintained for at least a short time period to allow the time trace of the measurand to be acquired.

Thus, the selected sensor is uniquely aligned with the selected interrogation wavelength at a single read-out time point, but the selected sensor may further be maintained to be uniquely aligned with the interrogation wavelength during a time period for acquiring a time trace of the measurand from the selected sensor.

Each sensor may be configured to generate a time-varying modulation of the optical interrogation signal by receiving a time-varying measurand signal. Thus, the time-varying measurand signal may be converted into a time trace of the measurand in the optical interrogation signal.

For instance, the sensor device may be used for photo-acoustic imaging. In such case, a pulsed laser is used to emit optical light pulses. The optical light pulses are converted into acoustic pulses upon absorption of the optical light pulses by a sample to be imaged. A total duration for recording the acoustic pulse can be of tens or hundreds of µs. During the measurement of the acoustic pulse , the selected sensor may be uniquely aligned with the selected interrogation wavelength in order to allow the time trace of a response to the photo-acoustic pulse to be acquired by the selected sensor. Then, other sensor(s) may be selected for measuring responses to later photo-acoustic pulses.

It should be realized that the sensor device provides for a systematic manner of reading out measurements from a plurality of sensors. The measurements may be read out using time multiplexing and wavelength multiplexing in the optical interrogation signal. Thanks to the systematic manner of controlling sensors to be uniquely aligned with selected interrogation wavelengths, the time multiplexing and wavelength multiplexing may be efficiently used for read-out of measurements from the plurality of sensors.

The sensor device may be associated with a single detector for detecting the optical interrogation signal after the optical interrogation signal has interacted with the selected sensors. The single detector may be configured to demultiplex the optical interrogation signal, e.g., by separating the optical interrogation signal into separate signal components with different interrogation wavelengths. The single detector may thus comprise a plurality of photodetectors for detecting different interrogation wavelengths.

The detector may be configured to detect an intensity of incident light on the detector. For instance, the plurality of photodetectors may be used for detecting intensity of light with respective interrogation wavelengths. The intensity of incident light may provide a representation of modulation of the optical interrogation signal by the measurand.

The detector may be configured to receive a transmitted portion of the optical interrogation signal. If an optical resonance translates into a minimum of transmission and the interrogation wavelength of the signal component corresponds well with the optical resonance wavelength, an intensity of the signal component of the optical interrogation signal reaching the detector is therefore low. If an optical resonance translates into a maximum of transmission and the interrogation wavelength of the signal component corresponds well with the optical resonance wavelength, an intensity of the signal component of the optical interrogation signal reaching the detector is therefore high. The detector may alternatively be configured to receive a reflected, absorbed, or scattered portion of the optical interrogation signal. Again, if an optical resonance translates into a maximum/minimum of reflection, absorption, or scattering, and if the interrogation wavelength of the signal component corresponds well with the optical resonance wavelength, an intensity of the signal component of the optical interrogation signal is therefore high/low, respectively.

It should be further realized that the sensor may be adapted to detecting a particular frequency. For instance, the sensor may comprise a membrane having a resonance frequency such that the membrane may be particularly sensitive to a measurand exhibiting a corresponding frequency. It should be realized that the optical resonance element may be arranged on the membrane such that vibrations of the membrane may affect the optical resonance element to allow the measurand to affect the optical characteristics of the optical resonance element. However, the optical resonance element may be provided with a resonance wavelength independently of the resonance frequency of the membrane. Thus, the resonance wavelength of the optical resonance element is not necessarily tied to wavelength or frequency information of the measurand that is to be acquired by the sensor device.

According to an embodiment, the control element may be configured to cause a change of the resonance wavelength in a single direction, such as for increasing the resonance wavelength. Thus, the control element may be configured to apply a control of the optical resonance element of the sensor associated with the control element for increasing the resonance wavelength. The applied control may then be relaxed allowing the resonance wavelength to be brought back towards natural resonance wavelength.

Aligning of the resonance wavelength with a selected interrogation wavelength may include controlling the resonance wavelength by the control element to be increased above the selected interrogation wavelength and thereafter allowing the resonance wavelength to be reduced for bringing the resonance wavelength to match the selected interrogation wavelength. This may be beneficial in order to avoid issues related to the self-heating of the optical resonance element based on the interrogation wavelength being coupled into the optical resonance element to a higher degree as the resonance wavelength is brought towards the interrogation wavelength by increasing the resonance wavelength. The self-heating may cause the resonance wavelength to be increased such that the resonance wavelength further increases by the self-heating effect as the resonance curve matches the selected interrogation wavelength to an increasingly better extent. By bringing the resonance wavelength towards the selected interrogation wavelength by the resonance wavelength being decreased towards the selected interrogation wavelength, the resonance wavelength may be more easily aligned at an optimum setpoint to the selected interrogation wavelength and the aligning of the resonance wavelength with the selected interrogation wavelength is not affected by the self-heating effect.

It should be realized that the resonance wavelength of the optical resonance element may be dependent on an intensity of a control signal provided by the control element. Thus, if the control element is configured to apply the control signal for increasing a resonance wavelength of the optical resonance element, a decrease in intensity of the control signal may cause the resonance wavelength to be decreased. Thus, the control signal may need to be continuously held at a constant level for ensuring that the resonance wavelength is held at a constant desired value. The applied control may thus be relaxed for bringing the resonance wavelength to be brought back towards natural resonance wavelength by an intensity of a control signal provided by the control element being reduced.

According to an embodiment, each of the control elements comprises a heater for heating the optical resonance element of the sensor associated with the control element, wherein the heater is configured to be controlled, for aligning the resonance wavelength with the selected interrogation wavelength, to heat the optical resonance element to change the resonance wavelength from the at least one wavelength range to a wavelength larger than the selected interrogation wavelength and thereafter cool the optical resonance element to bring the resonance wavelength to match the selected interrogation wavelength.

The heater may provide a simple manner of controlling the resonance wavelength of the optical resonance element of the sensor. The heater may be configured to efficiently change the resonance wavelength based on a control signal provided to the heater.

The heater may be configured to provide heating of the optical resonance element to control a temperature of the optical resonance element for controlling the resonance wavelength. The optical resonance element may thus be brought to a temperature above a temperature of surroundings to the optical resonance element. This implies that if an intensity of heating by the heater is reduced, the temperature of the optical resonance element may be reduced. Hence, the optical resonance element may be cooled by reducing the intensity of heating by the heater. Thus, during alignment of the resonance wavelength with the selected interrogation wavelength, the heater may initially be controlled to provide heating at a high intensity and may thereafter be controlled to provide heating at a reduced intensity to allow the optical resonance element to be cooled for bringing the resonance wavelength to match the selected interrogation wavelength. Thus, at a set point to be used for alignment of the resonance wavelength with the selected interrogation wavelength, the heater may provide heating at a moderate intensity to continuously maintain the resonance wavelength aligned with the selected interrogation wavelength.

According to an embodiment, the sensor device further comprises a detector configured to detect the at least one interrogation wavelength, wherein the detector is configured to detect intensity of the at least one interrogation wavelength during alignment of the resonance wavelength of the selected sensor with the selected interrogation wavelength for identifying a set point for alignment.

Thus, the detector may be configured to detect the selected interrogation wavelength of the optical interrogation signal. When the selected sensor is aligned with the resonance wavelength, intensity of the optical interrogation signal at the interrogation wavelength may be affected, such that the detector may provide an indication when alignment is reached. Hence, the detector may be used for identifying the set point for alignment to ensure that the selected sensor is properly aligned with the selected interrogation wavelength before measurements are performed.

The use of the detector may thus ensure that the resonance wavelength of the selected sensor is properly aligned with the selected interrogation wavelength. This may be particularly useful to ensure that the resonance wavelength of the selected sensor is aligned with the selected interrogation wavelength in external conditions that may further affect the resonance wavelength.

However, according to an alternative, the sensor device may comprise look-up tables providing information of control signals to be used for respective sensors for aligning the resonance wavelength of the selected sensors with the selected interrogation wavelengths. The look-up tables may provide information based on calibration of the sensor device.

The use of look-up tables may provide a very fast aligning of the resonance wavelengths of the selected sensors with the selected interrogation wavelengths, since proper intensities of control signals to the control elements may be immediately applied. However, the look-up tables may for instance not be able to handle changes to the resonance wavelengths based on changes in external conditions. Thus, use of a detector may be preferred.

The detector may also be configured to detect the optical interrogation signal for detecting measurement information carried by the optical interrogation signal. Thus, the detector may be used both for detecting the measurement information and also for detecting information for allowing the resonance wavelengths of the selected sensors to be aligned with the interrogation wavelengths before read-out of measurements.

According to an embodiment, the detector may comprise a splitter for separating different interrogation wavelengths of the optical interrogation signal and a plurality of photodetectors, each photodetector being configured to receive light of a unique interrogation wavelength being separated by the splitter.

Thus, the optical interrogation signal may be separated so as to separate different wavelength components of the optical interrogation signal. Each interrogation wavelength may then be simply detected by a photodetector. This is a simple manner of demultiplexing the information carried by the plurality of interrogation wavelengths of the optical interrogation signal.

The splitter may for instance be provided by an arrayed waveguide grating (AWG). The AWG may be useful for providing an accurate splitting of the interrogation wavelengths of the optical interrogation signal.

It should be further realized that the detector may not necessarily detect the optical interrogation signal that is propagated by the waveguide. According to an alternative, the optical interrogation signal may cause a coupling of light via the optical resonance element to an additional waveguide in which a detection signal based on the optical interrogation signal may be formed. The detection signal may comprise light at the interrogation wavelengths since this light may be coupled from the optical interrogation signal via the optical resonance elements to the additional waveguide. Hence, the detector being configured to detect the at least one interrogation wavelength may imply that the detector detects a detection signal based on the optical interrogation signal and not necessarily that the detector detects the optical interrogation signal.

According to an embodiment, the sensor device is configured for the resonance wavelength of each of the sensors in the group of sensors, before read-out of measurements from the group of sensors, being in the at least one wavelength range, by the sensor device being configured to provide control signals to the heater of each of the control elements for controlling the resonance wavelength of the sensor associated with the control element to be tuned to the at least one wavelength range.

Thus, the heaters may provide pre-heating for controlling the resonance wavelengths for non-selected sensors to be set to the at least one wavelength range. This implies that the resonance wavelengths of the non-selected sensors may be accurately defined. Hence, the at least one wavelength range, or storage window, may have a small width.

According to another embodiment, the sensor device is configured for the resonance wavelength of each of the sensors in the group of sensors, before read-out of measurements from the group of sensors, being in the at least one wavelength range, by the sensor device being manufactured with tolerances ensuring that a nominal resonance wavelength of each sensor is within the at least one wavelength range.

Thus, the sensor device may be manufactured with suitable tolerances for ensuring that each sensor is nominally within the at least one wavelength range. This may imply that no control signals may need to be applied to non-selected sensors, such that power consumption associated with non-selected sensors may be minimized.

According to an embodiment, the sensor device is configured to, during alignment of the resonance wavelength of the selected sensor with the selected interrogation wavelength, modulate a control signal to the control element to which the selected sensor is associated or modulate the interrogation wavelength, using a modulation frequency.

This may be used for easily finding an optimal set point for alignment of the resonance wavelength with the selected interrogation wavelength. The detector may be configured to detect an intensity modulation of the optical interrogation signal based on the modulation frequency. This implies that lock-in detection may be used for detecting amplitude of the intensity modulation, wherein a maximum detected amplitude implies that the resonance wavelength of the selected sensor is aligned with the selected interrogation wavelength at a set point of maximum sensitivity of the sensor.

It should be further realized that modulation of control signals to a plurality of control elements may be used for simultaneously aligning resonance wavelengths of a plurality of selected sensors with respective interrogation wavelengths. Control signals of different control elements may then be associated with different modulation frequencies. Thus, by detecting the modulation frequency associated with a specific interrogation wavelength, it is possible to determine which sensor is aligned with the respective interrogation wavelengths.

According to another embodiment, the sensor device may be configured to detect thermomechanical noise in the optical interrogation signal while varying the control signal to the control element of the selected sensor. A noise time trace of a sensor may be dominated by thermomechanical noise. The detected noise may be used for identifying maximal variance in the thermomechanical noise corresponding to a set point of maximum sensitivity of the sensor.

According to an embodiment, the optical interrogation signal comprises a plurality of separate interrogation wavelengths, wherein the sensor device is configured for simultaneous read-out of measurements from a set of selected sensors among the group of sensors, wherein, for each of the selected sensors, the sensor device is configured to tune the resonance wavelength to match a unique interrogation wavelength of the plurality of separate interrogation wavelengths.

Thus, thanks to the optical interrogation signal comprising a plurality of separate interrogation wavelengths, measurements from a plurality of selected sensors may be read out simultaneously. This implies that high speed of read-out of measurements from all of the plurality of sensors of the sensor device is facilitated.

According to an embodiment, the sensor device is configured to align a first resonance wavelength of a first selected sensor to a first interrogation wavelength and to align a second resonance wavelength of a second selected sensor to a second interrogation wavelength, wherein the first interrogation wavelength is offset farther from the at least one wavelength range than the second interrogation wavelength, and wherein the sensor device is configured to align the first resonance wavelength to the first interrogation wavelength before aligning the second resonance wavelength to the second interrogation wavelength.

Thus, the sensor device may be configured to control one selected sensor at a time for aligning the resonance wavelength of the respective sensors to interrogation wavelengths. This implies that the aligning of the resonance wavelength of the selected sensor to an interrogation wavelength will immediately provide information of which interrogation wavelength that the selected sensor is associated with.

It should be realized that, during alignment of the second sensor, the resonance wavelength of the second sensor may be increased past interrogation wavelengths. When the resonance wavelength passes the interrogation wavelength, light with the interrogation wavelength may be coupled into the optical resonance element of the second sensor, such that intensity of light at the interrogation wavelength in the optical interrogation signal propagated by the waveguide decreases. If a resonance wavelength of the first sensor is already aligned with such interrogation wavelength, the alignment may be lost since intensity at the interrogation wavelength may decrease causing self-heating of the optical resonance element of the first sensor to decrease such that the resonance wavelength of the first sensor may also decrease.

Hence, thanks to the first selected sensor, for which alignment is performed first, being associated with a first interrogation wavelength being offset farther from the storage window than the second interrogation wavelength, the aligning of the resonance wavelength of the second sensor will not affect the alignment that has already been performed for the first sensor.

It should be realized that alignment may be performed sequentially for a plurality of selected sensors to the plurality of interrogation wavelengths, wherein the sensors are aligned sequentially to selected interrogation wavelengths having a decreasing offset from the at least one wavelength range in the sequence. This may ensure that alignment of the plurality of selected sensors may be performed such that alignment is maintained for already aligned sensors during the alignment procedure.

According to another embodiment, the sensor device is configured to align a first resonance wavelength of a first selected sensor to a first interrogation wavelength and to align a second resonance wavelength of a second selected sensor to a second interrogation wavelength, wherein the first selected sensor is closer than the second selected sensor to an inlet port of the waveguide at which the optical interrogation signal is received into the waveguide, and wherein the sensor device is configured to align the first resonance wavelength to the first interrogation wavelength before aligning the second resonance wavelength to the second interrogation wavelength.

Thus, alignment of the resonance wavelengths of selected sensors may be performed sequentially for a plurality of selected sensors in an order in which the selected sensors are arranged along the waveguide. It should be realized that if intensity of the first interrogation wavelength is affected by the second sensor, the first selected sensor is not affected, since the optical interrogation signal is then affected in a location of the waveguide after the optical interrogation signal has already passed the first selected sensor.

This is another manner to ensure that alignment of the plurality of selected sensors may be performed such that alignment is maintained for already aligned sensors during the alignment procedure.

According to an embodiment, the sensor device is configured for arranging the resonance wavelength of each of the sensors, before read-out of measurements from the sensors, in a plurality of distinct wavelength ranges, wherein the plurality of separate interrogation wavelengths comprises a plurality of sets of multiple interrogation wavelengths, each set of interrogation wavelengths being associated with a unique wavelength range.

Thus, the at least one wavelength range may comprise a plurality of distinct wavelength ranges. Hence, the sensor device may utilize a plurality of storage windows in which the resonance wavelengths of non-selected sensors are arranged.

Thank to having a plurality of distinct wavelength ranges, a wavelength shift required for bringing a resonance wavelength to be aligned with an interrogation wavelength may be relatively small. Thus, if heating is used for controlling the resonance wavelength, a required heating power for bringing the resonance wavelengths of sensors in alignment with the interrogation wavelengths may be relatively small. Hence, the use of a plurality of distinct wavelength ranges may be beneficial for providing a low power consumption by the control elements.

However, the use of the plurality of distinct wavelength ranges implies that the wavelength ranges may fill a relatively large portion of a wavelength spectrum, such that a number of interrogation wavelengths that may be used may be decreased (compared to when fewer wavelength ranges are used). Hence, if the number of interrogation wavelengths need to be maximized, a single wavelength range (storage window) may be used.

Each set of multiple interrogation wavelengths may be arranged in a specific wavelength range. Hence, the wavelength range of the multiple interrogation wavelengths may be next to the wavelength range (storage window) with which the set is associated. This further implies that between two of the distinct wavelength ranges (storage windows), a single set of multiple interrogation wavelengths is provided.

According to an embodiment, the sensor device is configured for controlling read-out of measurements from a plurality of sets of selected sensors among the group of sensors, wherein the sensor device is configured to time-multiplex read-out of measurements from different sets of selected sensors.

This implies that the group of sensors with which the waveguide is associated may comprise a larger number of sensors than a number of interrogation wavelengths within the optical interrogation signal. Thus, all of the sensors in the group of sensors may not be read out at the same time. This is possible thanks to resonance wavelengths of non-selected sensors being arranged to not interfere with the optical interrogation signal and thanks to the possibility of dynamically changing the resonance wavelengths for selecting sensors for read-out.

Still, the sensor device may be able to read out measurements from all of the sensors in the group of sensors using time-multiplexing.

The waveguide may be provided with inlets and outlets that require some space. Thanks to the sensor device enabling a single waveguide to be associated with a large number of sensors, arrangement of inlets and outlets of the waveguide(s) need not affect a compactness of the plurality of sensors in the sensor device. For instance, a single waveguide may be associated with an entire row of sensors in an array, such that the inlets and outlets of the waveguide may be provided in an area outside an area of the array.

According to an embodiment, the sensor device is configured to control selected sensors within different sets to be physically arranged interlaced with each other.

The sensor device may be configured to read out information from all sensors in a set simultaneously. Thus, different sets are read out at different time points using the time-multiplexing.

During read-out of information, the control elements of the selected sensors may be active. For instance, if control elements are implemented by heaters, heating is provided to the selected sensors in a set simultaneously. The heating from a control element associated with one selected sensor may also cause slight heating of a neighboring sensor. This implies that there may be crosstalk between the control of adjacent control elements.

However, by arranging selected sensors within different sets interlaced with each other, closest neighboring sensors to a selected sensor are not read out simultaneously with the selected sensor. This implies that an effect of crosstalk between control elements of selected sensors within a set is reduced, allowing alignment of the resonance wavelengths of the selected sensors with the selected interrogation wavelengths to be accurately controlled.

According to an embodiment, the plurality of sensors is arranged on a common substrate and wherein the substrate comprises trenches between neighboring sensors.

The trenches may be used for reducing thermal crosstalk between neighboring sensors. This may ensure that control of the resonance wavelength provided by the control elements of one sensor does not affect the resonance wavelengths of neighboring sensors.

The trenches may provide poor thermal conductivity. The trenches may be filled by a solid material having poor thermal conductivity, such as silicon oxide.

According to an embodiment, the trenches also provide poor acoustic conductivity. The trenches may be open, i.e., may not be provided with any solid material. For instance, air, a vacuum, or water may be provided in the trenches. This implies that the sensors may be arranged such that acoustic crosstalk between neighboring sensors may be avoided. This may be particularly suitable for a sensor device, wherein the measurand to be measured is an acoustic wave.

The sensor device may comprise separate trenches, such that a pair of sensors is associated with a unique trench arranged between the sensors in the pair. The sensor device may comprise a plurality of trenches, wherein each trench is arranged between two neighboring sensors in a row of sensors. Thus, trenches may be arranged between each pair of sensors in the row of sensors.

This implies that trenches may be provided to avoid crosstalk between sensors, while the sensor device still allows structures to be arranged to extend across a plurality of sensors. For instance, the sensor device may comprise a plurality of waveguides, wherein each waveguide extends along a row of sensors. The waveguide may thus extend between trenches associated with different rows.

There may not be a need to have trenches between sensors arranged in different rows of sensors, if waveguides extend along the rows of sensors. The sensors in different rows may be read out using different waveguides and the sensor device may provide time-multiplexing between different waveguides, such that any thermal crosstalk between rows will not affect active sensors.

According to an embodiment, the plurality of separate interrogation wavelengths is within a free spectral range of optical resonance elements of the plurality of sensors.

Each optical resonance element may have a set of resonance wavelengths. The resonance wavelengths correspond to modes of different order. A free spectral range of an optical resonance element corresponds to a difference in wavelengths between the resonance wavelengths of different modes that differ by 1 when the resonances are sequentially numbered in the transmission curve.

Thanks to the interrogation wavelengths being within the free spectral range of the optical resonance elements, higher and lower harmonics of the resonance wavelengths will not interfere with the interrogation wavelengths.

Thus, where a plurality of separate interrogation wavelengths is used in the optical interrogation signal, all of the interrogation wavelengths are preferably arranged within the free spectral range of the optical resonance elements. This implies that the aligning of the resonance wavelengths with the selected interrogation wavelengths may be accurately controlled without any interference being caused by lower or higher harmonics of the resonance wavelengths affecting non-selected interrogation wavelengths.

According to an embodiment, the sensor device is configured for controlling read-out of measurements from a plurality of groups of sensors, wherein the sensor device is configured to time-multiplex read-out of measurements from different groups of sensors.

Thus, the sensor device may comprise a plurality of waveguides, each associated with a different group of sensors among the plurality of groups. For instance, each waveguide may be associated with a unique row of sensors in an array.

The time-multiplexing of read-out of measurements imply that only a single waveguide may need to be used at a time. This further implies that a single light generating unit may be provided for generating the optical interrogation signal. This single light generating unit may be shared by the plurality of waveguides, such that the optical interrogation signal is coupled into the waveguides in a time-multiplexed manner.

Similarly, a single detector may be used for detecting the optical interrogation signal. The single detector may be shared by the plurality of waveguides, such that the waveguides are connected to the detector in a time-multiplexed manner.

According to an embodiment, the sensor device is configured to tune the resonance wavelength of the selected sensor for aligning the resonance wavelength with the selected interrogation wavelength by the selected interrogation wavelength being arranged at a flank of a resonance curve of the resonance wavelength.

This is an efficient manner of sensing the measurand with high sensitivity, since a slight change in the resonance wavelength, as caused by the measurand, may provide a large difference in modulation of optical intensity of the optical interrogation signal.

According to an embodiment, the waveguide forms part of a plurality of waveguides, wherein each waveguide is associated with a unique group of sensors, wherein a group of waveguides among the plurality of waveguides are configured to simultaneously receive the optical interrogation signal for simultaneous read-out of measurement from selected sensors from the groups of sensors associated with the group of waveguides.

This enables a light generating unit to be shared by the plurality of waveguides. In addition, each waveguide within a group of waveguides may be associated with a unique photodetector for detecting the optical interrogation signal from the waveguide associated with the photodetector. Hence, light generating unit and photodetectors may be efficiently used.

Further, the possibility of controlling the resonance wavelengths of the selected sensors ensures that different sensors associated with a particular waveguide may be controlled to be read out at different time points.

The optical interrogation signal may comprise only a single interrogation wavelength. This may imply that one sensor may be read out from each waveguide at a time. However, the optical interrogation signal may alternatively comprise a plurality of interrogation wavelengths allowing a plurality of sensors per waveguide to be read out simultaneously.

The light generating unit may comprise a tunable laser such that the optical interrogation signal may be tuned between different read-out time points for setting the interrogation wavelength to different wavelengths at different time points. Thus, the optical interrogation signal may be changed in time to be adapted to the resonance wavelengths of different sensors. The control elements may be used for controlling the resonance wavelength of the selected sensor to be precisely aligned to the interrogation wavelength to ensure accurate read-out of measurements.

According to a second aspect, there is provided a photo-acoustic imaging device comprising: the sensor device according to the first aspect, wherein each sensor of the plurality of sensors comprises an opto-mechanical element for receiving an acoustic wave forming the measurand, and wherein the opto-mechanical element is configured to be affected by the received acoustic wave so as to change the optical characteristic of the optical resonance element.

Effects and features of this second aspect are largely analogous to those described above in connection with the first aspect. Embodiments mentioned in relation to the first aspect are largely compatible with the second aspect.

The use of the sensor device in the photo-acoustic imaging device enables fast and simultaneous read-out of a plurality of sensors of the sensor device.

This implies that the photo-acoustic imaging device may be used, e.g., for real-time monitoring of an object using photo-acoustic imaging. Photo-acoustic imaging may involve using optical pulses to excite acoustic signals in a sample, such as a liquid, gas, solid, tissue. The acoustic signal may then be detected by the plurality of sensors for enabling imaging of the sample.

The photo-acoustic imaging device may use a large number of sensors, enabling imaging of the object with high resolution or imaging of a large size object, while numerous sensors may be read out simultaneously to allow fast updates of images of the object.

The photo-acoustic imaging device comprises opto-mechanical elements for receiving an acoustic wave, wherein the acoustic wave affects an optical characteristic of the optical resonance element such that the acoustic wave is detected.

The opto-mechanical elements may be implemented in various manners. For instance, the opto-mechanical elements may comprise a mechanical element that may be formed by membranes, such that each sensor comprises a membrane for receiving an acoustic wave forming the measurand, wherein the membrane is configured to vibrate in dependence of the received acoustic wave, whereby an optical characteristic of the optical resonance element arranged on or in the vicinity of the membrane may be affected. According to an alternative, the opto-mechanical element may comprise a disk configured to bend upon receiving an acoustic wave or a Fabry-Pérot sensor.

According to a third aspect, there is provided a method for read-out of a detected measurand, said method comprising: receiving an optical interrogation signal comprising at least one interrogation wavelength in a waveguide associated with a group of sensors among a plurality of sensors, wherein each sensor comprises an optical resonance element, wherein an optical characteristic of the optical resonance element is configured to be affected by the measurand to be measured by the sensor, wherein, before read-out of measurements from the group of sensors, the resonance wavelengths of each of the sensors in the group of sensors is in at least one wavelength range, wherein the at least one interrogation wavelength is outside the at least one wavelength range; and tuning the resonance wavelength of a selected sensor by controlling a control element to which the selected sensor is associated for aligning the resonance wavelength with a selected interrogation wavelength of the at least one interrogation wavelength; and reading out the detected measurand from the selected sensor by the optical interrogation signal, wherein the selected sensor is during read-out uniquely aligned with the selected interrogation wavelength for reading out the measurand.

Effects and features of this third aspect are largely analogous to those described above in connection with the first and second aspects. Embodiments mentioned in relation to the first and second aspects are largely compatible with the third aspect.

According to the method, read-out of the information carried in resonance wavelengths of a plurality of sensors is provided in a systematic manner, enabling fast read-out of the measurand from the plurality of sensors while ensuring that read out information can be unambiguously tied to a particular sensor in the plurality of sensors.

The method enables read-out of information from the plurality of sensors in such manner that the plurality of sensors may be easily scaled, i.e., provided with a huge number of sensors, while the read out of information may still be provided in a fast manner and without requiring extensive hardware (such as numerous light sources providing multiple interrogation wavelengths and/or numerous detectors for detecting optical interrogation signals).

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1a is a schematic perspective view of a sensor according to an embodiment with a partially cut-away membrane to illustrate all features of the sensor.
Fig. 1b is a schematic top view of the sensor in Fig. 1a with a cut-away membrane.
Fig. 1c is a schematic graph illustrating detection of intensity changes of a transmitted signal based on a measurand acting on the sensor.
Fig. 2a is a schematic graph illustrating a transmission curve of a sensor and a self-thermo-optic effect of altering the transmission curve of the sensor.
Fig. 2b is a schematic graph illustrating a hysteresis behavior of the transmission curve in relation to an increasing or decreasing wavelength of light of a read-out laser.
Fig. 3 is a schematic graph illustrating a set point of sensitivity of a sensor in relation to the transmission curve.
Fig. 4a is a schematic view of a sensor device illustrating a multiplexing scheme allowing reading out measurements only from a limited number of sensors.
Fig. 4b is a schematic graph illustrating the limited number of sensors that may be read out using the multiplexing scheme of the sensor device of Fig. 4a.
Fig. 5 is a schematic view illustrating arrangement of inputs and outputs in relation to an array of sensors of a sensor device according to an embodiment.
Fig. 6 is a schematic view of a portion of a row of sensors according to an embodiment and a graph illustrating control of resonance wavelengths of the sensors.
Fig. 7a is a schematic view of a sensor device according to an embodiment.
Fig. 7b is a flow chart of a method for reading out measurements from a plurality of sensors.
Fig. 8a is a schematic view of sequential graphs illustrating a change of resonance wavelength during alignment of the resonance wavelength of a sensor with an interrogation wavelength of an optical interrogation signal.
Fig. 8b is a schematic graph of a measured intensity of the optical interrogation signal during the alignment process shown in Fig. 8a.
Fig. 9 is a schematic graph of a control signal to a heater used for alignment of the resonance wavelength with the interrogation wavelength.
Fig. 10a is a schematic graph of arrangement of resonance wavelengths of a plurality of sensors before and during read-out of measurements according to a first embodiment.
Fig. 10b is a schematic graph of arrangement of resonance wavelengths of a plurality of sensors before and during read-out of measurements according to a second embodiment.
Fig. 11 is a schematic view of graphs illustrating an effect of a spectral spread of interrogation wavelengths on cross-talk between sensors.
Fig. 12 is a schematic graph of arrangement of resonance wavelengths of a plurality of sensors before and during read-out of measurements according to a third embodiment.
Fig. 13a is a schematic graph illustrating a spread of resonance wavelengths of sensors due to large fabrication imperfections.
Fig. 13b is a schematic graph of arrangement of resonance wavelengths of a plurality of sensors before and during read-out of measurements according to a fourth embodiment that is useful in a situation illustrated in Fig. 13a.
Fig. 14 is a schematic graph illustrating measurement of intensity of the optical interrogation signal during the alignment process for controlling the alignment process.
Fig. 15a is a schematic view of an arrangement of sensors in a row.
Fig. 15b is a schematic graph illustrating effect of an order of performing the alignment process for the sensors shown in Fig. 15a.
Fig. 16 is a schematic graph illustrating an order of aligning resonance wavelengths with a plurality of interrogation wavelengths.
Fig. 17 is a schematic graph illustrating a simultaneous aligning of a plurality of resonance wavelengths with a plurality of interrogation wavelengths.
Fig. 18a is a schematic view of sensors in a row illustrating a time-multiplexing scheme according to a first embodiment for reading out measurements from the sensors.
Fig. 18b is a schematic view of sensors in a row illustrating a time-multiplexing scheme according to a second embodiment for reading out measurements from the sensors.
Fig. 19 is a schematic view of part of an array of sensors illustrating trenches between sensors for reducing thermal cross-talk.
Fig. 20 is a schematic view of a sensor device according to a second embodiment.
Fig. 21 is a schematic view of a sensor device according to a third embodiment.

### Detailed description

According to the first aspect, there is provided a sensor device adapted for read-out of measurements from a plurality of sensors. The sensor device will be described below in relation mainly to measurements of a measurand being formed by an acoustic wave. However, it should be realized that the sensor device may be used for read-out of measurements of other types of measurands.

The use of optical Micro and Nano-Electro-Mechanical Systems (MOEMS and NOEMS) sensor devices, where the measurand measurement is carried out through an optical readout, is becoming widespread. Such sensor devices have multiple sensing applications such as chemical, mass, biological, pressure, temperature, force, and stiffness sensing.

The sensor devices comprise an optical resonance element, such as a ring, disk, sphere, Fabry Perot, or photonic crystals geometries. The measurand of interest modifies the sensor optical reflection or transmission response by changing a mechanical state of the sensor, which may be referred to as an optomechanical interaction.

Optomechanical ultrasound sensor (OMUS) devices 100 is an example of above discussed sensor devices and are used for ultrasound detection. Herein, read-out schemes will be discussed for OMUS devices. However, such schemes can be used for other MOEMS/NOEMS sensor device architectures and applications. In addition, the manner of multiplexed read-out of information could be generalized to other types of optical sensors, not necessarily based on the optomechanical interaction.

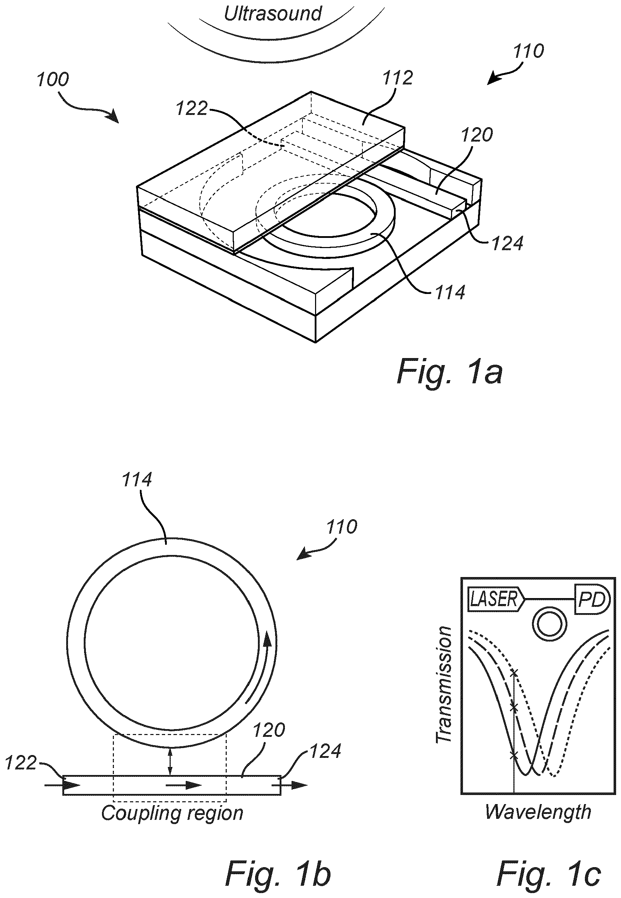

Referring now to Figs 1a-b, an OMUS pressure sensor 110 according to an embodiment may be based on a membrane 112 with a circular waveguide 114, or ring resonator forming an optical resonance element of the sensor 110, positioned below the membrane 112. The ring resonator 114 may be optically coupled to the membrane 112 such that an optical characteristic, e.g., a resonance wavelength, of the ring resonator 114 is changed in dependence of movement of the membrane 112. The ring resonator 114 may further be optically coupled to a straight waveguide 120. The straight waveguide 120 allows light forming an optical interrogation signal to be sent into the ring resonator 114 via an input port 122, and to transmitted intensity to be read out via a through port 124.

When the light coupled to the ring resonator 114 via the straight waveguide 120 is such that the optical path of the ring resonator 114 is a specific integer multiple of the wavelength, the ring resonator 114 is on resonance. The number of wavelengths m fitting in the optical path of the ring may be called the order of the mode. For light at the resonance wavelength of a confined mode in the ring resonator 114, destructive interferences occur between the light which has coupled to the ring resonator 114 and the light that passes directly through the straight waveguide 120. This implies that the total light intensity that passes through the straight waveguide 120 when the ring resonator 114 is at resonance decreases with respect to a case when the ring resonator 114 is off resonance. This results in a dip in a measured transmission curve at resonance.

As shown in Fig. 1c, when detecting pressure waves, such as acoustic waves, motion of the membrane 112 leads to changes in a gap between the ring resonator 114 and the membrane 112, which changes an effective refractive index of the mode confined in the ring resonator 114, which in turn changes the resonant wavelength of the ring resonator 114. The ultrasound pulse incident on the membrane 112 can be transduced into the optical interrogation signal propagating in the straight waveguide 120 by setting a read-out laser providing the optical interrogation signal to a flank of optical resonance of the ring resonator 114. As illustrated in Fig. 1c, the read-out laser may be set on the point of the flank where transmission slope is maximal, to maximize the optomechanical transduction sensitivity. Variations in optical intensities may be measured with a photodiode, enabling to acquire an electrical signal representing the measurand.

During photo-acoustic imaging, an object that is to be imaged is the source of the acoustic waves being measured. The object is excited by a pulsed laser, which is different from the read-out laser. The OMUS sensor device 100 may comprise a plurality of sensors 110 that are scanned to record multiple time traces from different sensors 110. These time traces may then be used to obtain a 3D image of the object.

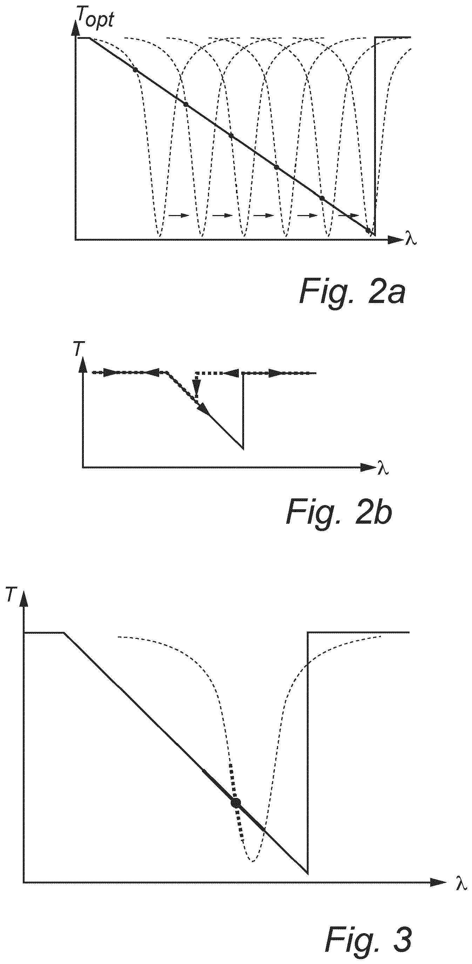

Referring now to Fig. 2a, the transmission curve of the OMUS sensor 110 has a Lorentzian shape that can be altered by a self-thermo-optic effect. The self-thermo-optic effect may occur when high input laser powers are coupled to the OMUS sensor 110 to readout the photo-acoustic signal. Indeed, when some of the light of the optical interrogation signal is absorbed, it causes an increase of temperature inside the ring resonator 114. As the refractive index of the ring resonator 114, which may for instance be formed by a semiconductor material, may depend on the temperature of the material (which is a thermo-optic effect), the effective refractive index of the confined optical mode in the ring resonator 114 depends on the coupled optical power. The effect is referred to herein as a self-thermo-optic effect to indicate that it is the optical interrogation signal, that is, the readout laser, which generates this effect.

When sweeping the transmission curve of a ring resonator 114 around one of its resonance wavelengths, the measured resonance curve is not Lorentzian, as further represented in Fig. 2a. Indeed, when tuning the read-out laser towards longer wavelengths (i.e., towards the right-hand side of Fig. 2a), the coupled power into the confined mode increases (solid line, Fig. 2a). This causes the effective refractive index to increase, and hence the resonance wavelength is increased, or red shifted, (successive Lorentzian curves, dashed lines, Fig. 2a). Hence, the optical mode moves away from a wavelength of the read-out laser, until the read-out laser is at the bottom of the optical mode (Lorentzian curve farthest to the right, Fig. 2a). In this case, an increase in the wavelength of the read-out laser decreases the coupled optical power, which cools down the mode, which further decreases the resonance wavelength, and so on. Hence the optical mode abruptly moves back to its initial spectral position before being heated by the laser (Lorentzian curve farthest to the left, Fig. 2a). This sequence of events results in a triangular shaped transmission curve.

In addition, transmission curves obtained when tuning the read-out laser towards longer wavelengths past the resonance wavelength of the ring resonator 114 and then tuning the read-out laser back towards shorter wavelengths present a hysteresis behaviour, as shown in Fig. 2b. When the laser sweeps towards longer wavelengths (i.e., optical mode interrogated from a left-hand side in Fig. 2a) the transmission curve looks like the curve represented by a solid line in Fig. 2b. When the laser sweeps towards shorter wavelengths, the transmission curve looks like the curve represented by a dashed line in Fig. 2b. This implies that if the optical mode is interrogated from the right-hand side, for a wavelength interval (i.e., the one where the hysteresis is visible, that is, when the curves are different depending on how we sweep the curve), the laser input wavelength can only couple to the optical mode in a limited wavelength range of the left flank of the optical resonance, reducing possibility of readout of the interrogated sensor 110, since this can prevent reaching the optimal setpoint of maximal sensitivity on the transmission curve.

Hence, it is clear that because of the triangular shape of the optical mode due to the self-thermo-optic effect, only the left flank of the transmission curve (i.e., wavelengths on the transmission curve shorter than a peak value of the transmission curve) is accessible to transduce optically the mechanical motion. Further, the ring resonator 114 is advantageously interrogated with the laser sweeping the transmission curve from the left hand-side (i.e., from shorter wavelengths towards longer wavelengths) of the resonance wavelength.

When dealing with an optomechanical sensor which is based on using a resonator, sensitivity of the sensor 110 depends on a slope at a set point on the flank of the optical resonance. Hence, an optimal set point needs to be reached to get maximal sensitivity of the optomechanical transduction. This means that a full flank of a dip in the transmission curve should be accessible for allowing an efficient alignment of an interrogation wavelength provided by the read-out laser to the optical resonance wavelength of the sensor 110.

Dynamics of mechanical displacements of the sensor 110 with respect to thermal constants of the optomechanical sensor 110 also need to be considered, as illustrated in Fig. 3. If characteristic frequencies f_{mech typical} of mechanical vibrations of the sensor 110 that are intended to be measured are much faster than a thermal time constant τ of the optomechanical sensor 110 (that is f_{mech typical} » 1/τ), then thermal dynamics of the optomechanical sensor 110 do not follow in time variations in optical intensity of an optical interrogation signal from the read-out laser being coupled into the ring resonator 114.

The optomechanical sensor 110 then behaves as if the temperature of the sensor 110 is constant, and the slope of the optical mode that influences a calculation of the sensitivity of the sensor is then controlled by the Lorentzian curve at the considered set point (dashed line in Fig. 3). If f_{mech typical} « 1/τ, then thermal dynamics of the optomechanical sensor 110 can follow the variations in optical intensity of the optical interrogation signal being coupled into the ring resonator 114. In this case, the slope of the triangular transmission curve based on the self-thermo-optic effect needs to be taken into account (solid line in Fig. 3) for determining sensitivity of the sensor 110.

It should be realized that the self-thermo-optic effect commonly occurs in the optical behaviour of the sensor 110 in NOEMS and MOEMS sensors which are based on optomechanical transduction, since the sensitivity of the sensor 110 is generally proportional to power of the optical interrogation signal from the read-out laser. This implies that the power may be increased for increasing sensitivity, leading to the operation of these sensors 110 in self-thermo-optic regime. Hence, developing readout schemes that account for the self-thermo-optic effect is desirable for real life applications, especially for sensors 110 fabricated in materials presenting strong thermo-optic coefficients such as silicon.

Since the length of the ring resonator 114 is linked specifically to resonant wavelengths of light, small fabrication defects in dimensions of fabricated sensors 110 can cause the resonance wavelengths to stochastically variate. To this end, some tuning may be needed in order to allow a specific wavelength of light to be used for interrogating the sensor 110.

The sensor 110 is associated with a control element 130 which is configured to control the resonance wavelength of optical resonance of the optical resonance element, such as the ring resonator 114, of the sensor 110. The control element 130 may be configured to change an optical characteristic of the ring resonator 114, such as changing a refractive index of the ring resonator 114.

The control element 130 may for instance be provided as a heater, which may be controlled by a small current. The heater may thus be configured to heat the ring resonator 114 such that the refractive index of the waveguide of the ring resonator 114 is changed due to the thermo-optic effect. This causes the resonance wavelength of the ring resonator to be shifted to a longer wavelength.

Thus, the alignment of the resonance wavelength of the ring resonator 114 with the interrogation wavelength provided by the read-out laser may be provided by controlling the resonance wavelength of the ring resonator 114 using the control element 130.

It should be realized that the sensor device 100 typically comprises a large plurality of sensors 110. Thus, capturing signals representing measurements coming from different sensors 110 is desired for the implementation of the sensor device 100 for real-life applications.

For example, in photo-acoustic imaging, multiple time traces may be recorded with ultrasound sensors 110 from multiple locations to reconstruct an image.

Another example is mass sensing based on optomechanical mass detectors. To this end, multiple mass landing events need to be recorded from highly sensitive and high frequency optomechanical mass sensors, to maximize a capture cross-section of the sensor device. This is particularly useful in biosensing and spectroscopy applications. Increasing the number of sensors decreases measurement time and a required quantity of analyte. In addition, it enables to get spatial mapping of the mass landing events.

To read, or multiplex/demultiplex, information of measurements coming from multiple MOEMS/NOEMS sensors 110, different degrees of freedom can be used. For instance, wavelength multiplexing may be used, wherein each sensor 110 is labelled with a unique readout interrogation wavelength. Hence, by coupling to each sensor a unique interrogation wavelength, and by the separating the interrogation wavelengths with a wavelength demultiplexer, the signals representing measurements originating from different sensors 110 may be retrieved. Additionally, or alternatively, time multiplexing may be used, wherein each sensor 110 is read sequentially in time. The sensor 110 may thus be identified by the time interval at which measurements are read out.

To increase the number of sensors 110 that can be interrogated, a multiplexing/demultiplexing scheme may be used in a controlled manner, for example by using the degrees of freedom described above. The read-out scheme should enable parallel (i.e., simultaneous) read-out of sensors 110, while preserving a simple measurement scheme. The read-out scheme should be scalable to a large number of sensors 110.

Hereinafter, a sensor device 100 exploiting the degrees of freedom providing efficient, systematic, and scalable read-out schemes that may be used with very large arrays of sensors 110 is described. The read-out schemes are provided, while preserving reasonable numbers of read-out lasers, photodiodes for detecting an optical interrogation signal having interacted with sensors 110, and fibres or waveguides for guiding optical signals. In addition, the sensor device 100 may provide a simple photonic integrated circuit (PIC) architecture providing guiding of the optical interrogation signal.

For instance, as mentioned above, in photo-acoustic imaging applications, read-out of a large array of optomechanical sensors 110 may be needed in a fast manner. The array comprises a plurality of OMUS sensors 110 that may be designed to respond to specific acoustic frequencies. The acoustic time traces recorded by the array of differently positioned sensors 110 allow reconstruction of photo-acoustic images.

Each OMUS sensor 110 can be identified by its row and column number in the array. If the array comprises P rows and M columns, then the total number of OMUS sensors 110 is equal to N = M × P. The number of sensors 110 to be read out can scale from tens up to thousands of sensors 110, depending on the application. The OMUS sensors 110 present in one row of the array may be evanescently coupled to the same waveguide 120, which may thus be associated with a group of sensors arranged in one row of the array. This provides a simple layout of the waveguide 120, which carries the optical interrogation signal to the sensors 110, in relation to the sensors 110.

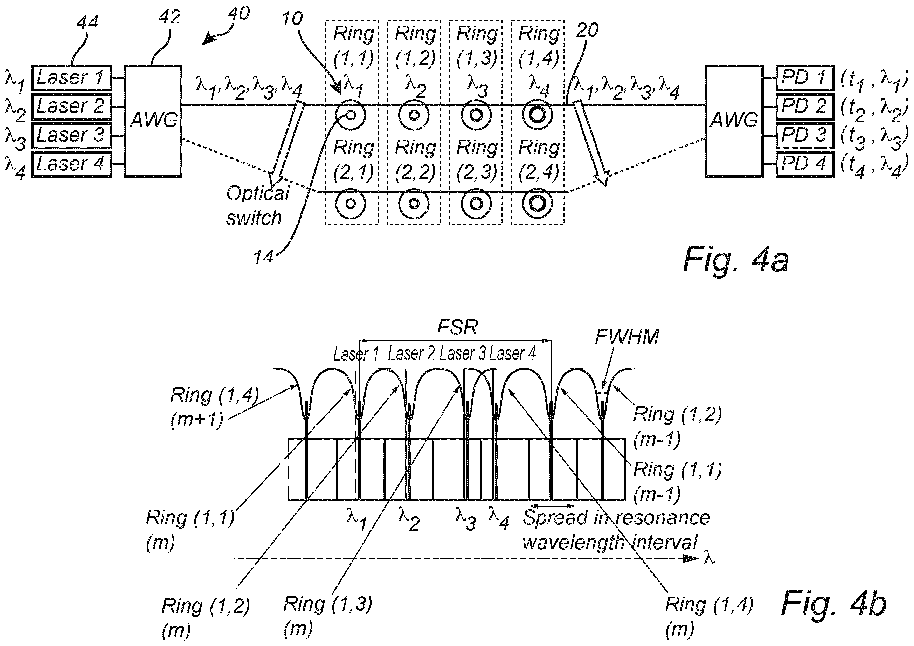

As shown in Fig. 4a, each ring resonator 14 may column-wise be fabricated with a slightly different diameter (size differences are exaggerated in Fig. 4a for illustration purposes), allowing to have a slightly different optical mode resonance wavelength for each sensor 10 on a given row.

To read out measurements from such an array of sensors 10, read-out schemes are needed that present realistic numbers of required read-out light sources, photodetectors, and simple PICs. One strategy is to implement read-out multiplexing schemes enabling to read out a row of the array using limited numbers of light sources and photo detectors.

An arrayed waveguide grating (AWG) component 42 and a bank of laser sources 44 providing different interrogation wavelengths may be used for creating a combined light source 40 providing an optical interrogation signal with multiple interrogation wavelengths. Each laser line is aligned with a different optical resonance wavelength of the rings on the row. After passing the sensors 10 evanescently coupled to the waveguide 20, the interrogation wavelengths can be split again and be individually measured.

Combining this wavelength multiplexing strategy with time multiplexing, an entire array of sensors 10 can be readout. An optical switch may be used for coupling the optical interrogation signal sequentially in time into waveguides 20 associated with different rows of the array, such that the optical interrogation signal is switched between consecutive rows once the measurement on a previous row is completed.

This wavelength multiplexing/demultiplexing scheme allows identifying each sensor 10 based on its associated resonance wavelength (column number) and time step (row number) at which it was measured.

However, wavelength multiplexing based on the configuration shown in Fig. 4a and discussed above presents fundamental limitations. Having many sensors 10 on a row, which are each to be identified with a unique interrogation wavelength, many read-out laser beams are needed each with a different wavelength that are spectrally adjacent to each other.

As shown in Fig. 4b, there is a limited wavelength interval available in which the interrogation wavelengths are to be provided, due to use of circular waveguides 14 providing an optical resonance element of the sensors 10. For example, if the diameter of the ring resonators 14 are increased over one row to increase the resonance wavelength of a confined mode of order m for each ring, the confined mode of order m for a ring will eventually reach a resonance wavelength of the confined mode of order m - 1 for a first ring in the row. A wavelength difference between the mode of order m and the mode of order m - 1 is called the free spectral range (FSR) of a ring. Thus, for each ring with a given resonance wavelength for the mode with a given order m, modes corresponding to higher and lower order values are also introduced (i.e., harmonics). Hence there is a limited wavelength interval in which the resonance wavelengths of the ring resonators should be placed. This wavelength interval is given by the FSR.

In addition, an optical mode of a ring resonator needs to be spectrally separated in the wavelength domain from optical modes of other ring resonators. The spectral space occupied by a resonance wavelength can be quantified for example by a full width half maximum (FWHM) of the mode. Hence, a limited number of different diameters of rings can be placed on an FSR.

Furthermore, the number of available interrogation wavelengths narrows even further when fabrication imperfections are considered, adding uncertainties on the resonance wavelength of the fabricated sensors 10. To illustrate this, the structure represented in Fig. 4a may be considered. In this case, since the diameter of ring resonator (1,1) is smaller than the diameter of ring resonator (1,2) and so on, identification of which mode belongs to which ring is provided by considering that the resonance wavelength λ₁ of ring resonator (1,1) is lower than the resonance wavelength λ₂ of ring resonator (1,2) and so on, that is λ₁ < λ₂ < ···. However, if there is a crossing between actual resonance wavelengths of two consecutive rings in the row, the demultiplexing may no longer correctly be used for identifying the respective sensors.

Hence, in order to ensure that which resonance belonging to which ring may be correctly identified a cleared window is needed. For example, assuming that the spread in the resonance wavelength follows a normal law, a 6σ cleared window (i.e., 3σ on each side), where σ is the standard deviation of the spread of the resonance wavelengths, ensures that the resonance wavelength of a sensor will fall within that window with a probability of 99.7%. Thus, only the resonance wavelength of a single ring resonator can be placed in this interval, as shown in Fig. 4b.

Statistics on OMUS sensors fabricated within a state-of-the-art clean room facilities show that the 6σ window may need to be of several nanometers. Further, the FSR of ring resonators 14 may be few tens of nanometers. Thus, it may be concluded that there are few separate windows available in the free spectral range (in the case of Fig. 4b, a situation with 4 windows fitted in the FSR are shown, for simplicity). Hence, only few sensors 10 can be placed in relation to a common waveguide 20 by following the implementation illustrated in Fig. 4a. Such number of sensors is too small in many applications, such as in photo-acoustic imaging. Hence, a different approach may be needed to enable combining more sensors on the same row to be read out using a single waveguide.

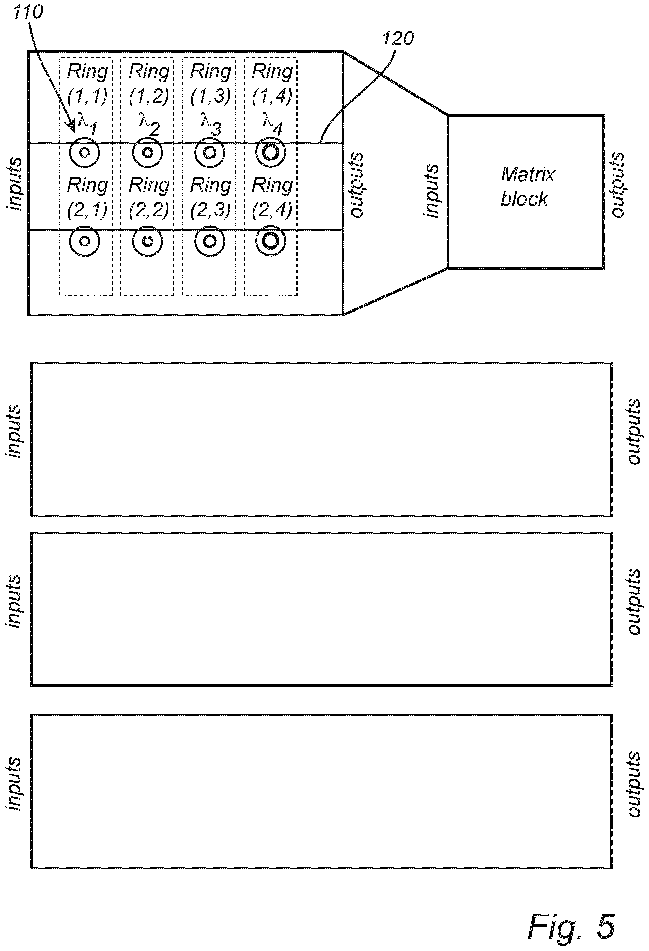

It may be preferable to have a single waveguide 120 associated with a row of sensors 110, as illustrated in Fig. 5. This implies that a PIC for routing optical signals in and out of the waveguide 120 may be relatively simply achieved, since inputs and outputs may be provided on opposite sides of the row. Thus, having a read-out scheme allowing implementation of a very large number of sensors 110 in the horizontal direction (that is along a row) is therefore desirable, as this enables a large array of OMUS sensors 110 to be provided, even if a pattern may need to be repeated in a vertical direction.

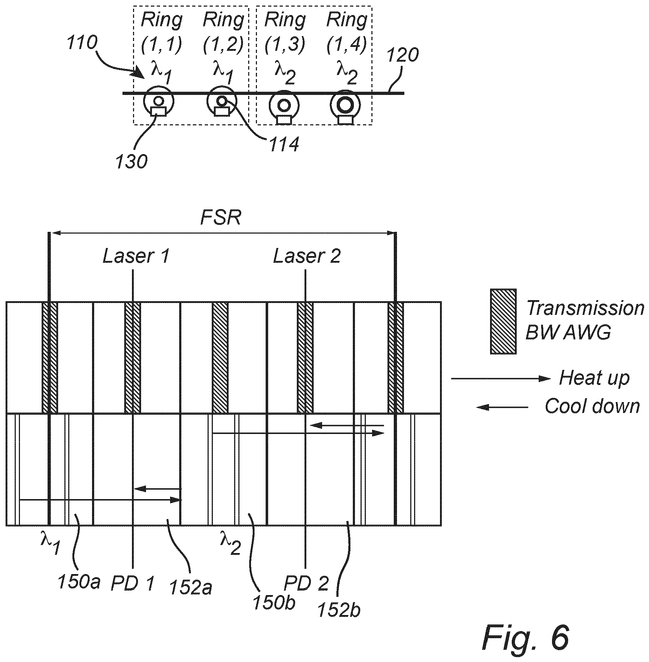

Referring now to Fig. 6, control of resonance wavelengths will be discussed. Using control elements 130, it is possible to dynamically (that is, over time) tune the resonance wavelength of the sensors 110. This can be provided by fabricating heaters 130 close to the sensors 110.

The heating provided by the heater 130 may be controlled during use of the sensor device 100 and can therefore be used for identifying a sensor 110 from neighboring sensors. It should be realized that other tuning mechanisms than using heating may be provided, such as mechanical-optical (e.g., piezoelectric, capacitive tuning) or electro-optical tuning mechanisms could alternatively be used for changing the resonance wavelength of the ring resonator 114.

To realize a systematic and scalable read-out scheme by using this degree of freedom, the spectral interval available, that is, the FSR of a ring resonator 114, may be subdivided into storage windows and readout windows.

The storage window(s) 150a, 150b provide one or more wavelength ranges that are used for "storing" all resonance wavelengths of the ring resonators 114 with a given nominal diameter when the sensors 110 are not used for read-out. The resonance wavelengths of the ring resonators 114 may be randomly spread within the storage window 150 because of the fabrication imperfections.

The readout windows 152a, 152b are associated with respective interrogation wavelengths (read-out lasers lines) that are used for reading the signals from the optomechanical sensors 110. Only one interrogation wavelength is present per readout window 152a, 152b. The interrogation wavelengths may be constant over time, which implies that widely tunable lasers are not needed for generating light at the interrogation wavelengths, and each laser line may be aligned with respect to transmission windows of an AWG. This allows use of standard light sources and AWGs.

The resonance wavelengths of the ring resonators 114 may be aligned with the interrogation wavelengths. The working principle of such a read-out scheme is represented in Fig. 6. To simplify, in this discussion in relation to Fig. 6, storage windows 150a, 150b and readout windows 152a, 152b having the same spectral widths are shown.

By heating a sensor 110 to bring the resonance wavelength of the sensor 110 from a storage window 150a, 150b to a readout window 152a, 152b read-out of that specific sensor may be selected and identified by knowledge of which heater 130 that is heated. This heating will cause the optical mode of the sensor 110 to be shifted towards a longer wavelength to bring the optical mode to the readout interrogation wavelength.

Increasing the temperature of the ring resonator 114, the thermo-optic effect can only tune the resonance wavelength to longer wavelengths for a ring resonator 114 made of silicon. Hence, the readout window 152a, 152b used for reading the modes of a given storage window 150a, 150b will in such case always be located on a right hand side in Fig. 6 (that is, at a longer wavelength) of the storage window 150a, 150b to which the interrogated ring resonator 114 belongs to.

Once a measurement is read out, the ring resonator 114 can be cooled down by turning off the heater 130. The mode returns back into the storage window 150a, 150b, and another sensor 110 can be pulled from the storage window 150a, 150b. This scheme can be repeated until all the modes (that is, all the sensors 110) in the storage window 150a, 150b are read out. Multiple ring resonators 114 belonging to different storage windows 150a, 150b can be read in parallel by different readout windows 152a, 152b, speeding up the readout of the row of sensors 110.

In Fig. 6, only 2 sensors were represented per storage window 150a, 150b (each sensor is represented by a solid vertical line in the storage window 150a, 150b). The number of sensors 110 per storage window 150a, 150b can be increased to a large number of sensors 110, all grouped in bundles of a ring resonators 114 with the same nominal diameter, meaning that k optical modes will be present with a very high probability in the storage window 150a, 150b. If b storage windows are placed over one FSR, then a × b ring resonators 114 can be placed per row of the array.

This implies that it is possible to fit way more sensors 110 onto the same row with respect to a case where the ring resonators 14 are non-tunable. This is possible by dynamically tuning the resonance wavelength of a selected sensor 110. When the measurement is finished for a sensor 110, the ring resonator 114 of the sensor 110 may be allowed to cool down and a next sensor 110 may be heated/activated. Naturally, this procedure can be implemented in parallel for all the storage windows 150a, 150b and readout windows 152a, 152b. This reduces the measurement time to the number of ring resonators 114 in the same storage window 150a, 150b times the measurement time per sensor 110. Hence, this opens a possibility of realistically providing long rows of ring resonators 114 all evanescently coupled with the same waveguide 120, allowing read-out of sensors 110 in large arrays to be provided with a layout as represented in Fig. 5.

The use of storage windows 150a, 150b and readout windows 152a, 152b present the advantage of providing an organized and systematic manner to arrange the optical resonance wavelengths and the interrogation wavelengths within FSR even though the resonance wavelengths of the optical modes are randomly distributed. In addition, the use of storage windows 150a, 150b and readout windows 152a, 152b allows ensuring that all the sensors 110 can be read out, even though in principle the thermo-optic effect may only enable tuning the resonance wavelengths in one direction, that is, towards higher values.

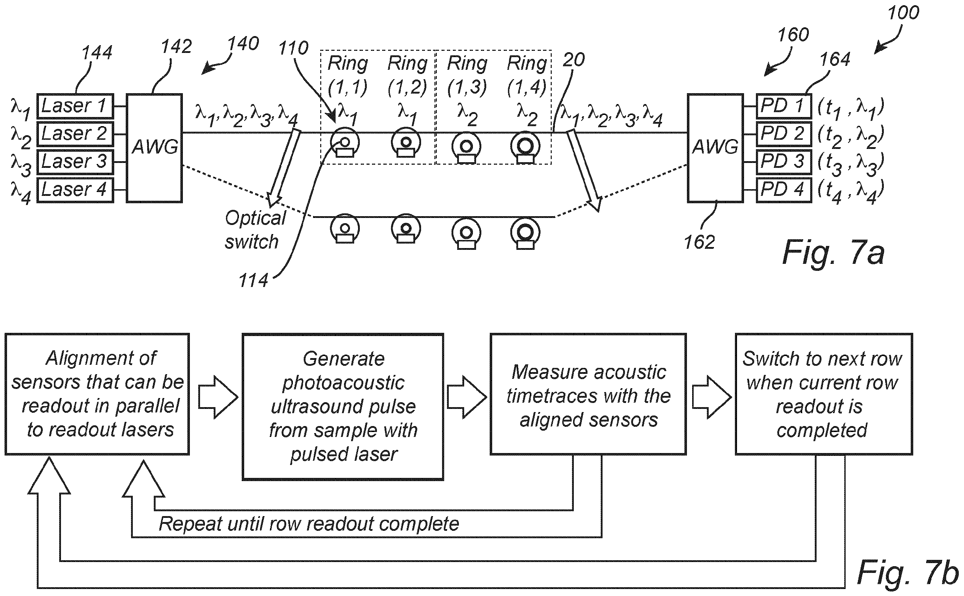

Fig. 7a illustrates arrangement of ring resonators 114, that may be individually controlled, in a 2D array layout. The ring resonators 114 in a row are grouped in storage windows 150a, 150b. The array of sensors 110 is connected to optical switches allowing time multiplexing read-out row by row. Fig. 7b illustrates a time sequence of read-out providing a read-out scheme.

The sensor device 100 may comprise a light source 140 comprising an AWG component 142 and a bank of laser sources 144 providing different interrogation wavelengths. The light source 140 may provide an optical interrogation signal comprising a plurality of interrogation wavelengths corresponding to the laser lines provided by the laser sources 144.

The sensor device 100 may further comprise a detector 160 comprising an AWG component 162 and a bank of photodiodes 164. The detector 160 may split the optical interrogation signal into different signal components, each having a unique interrogation wavelength, and the signal components may be detected by the bank of photodiodes 164.

Initially, the optical switches are aligned to a given row. The ring resonators 114 stored in storage windows 150a, 150b are aligned with their readout windows 152a, 152b. To this end, any storage/readout configuration presented hereinafter may be chosen. Once the alignment is completed, the pulsed laser is used for illuminating a sample and generating photoacoustic signals. The acoustic pulse time traces are measured with each aligned sensor 110 and saved. The alignment procedure ensures that it is known which sensor 110 that affects which signal component (interrogation wavelength) in the optical interrogation signal (i.e., multiplexing/demultiplexing). Once the measurement is finished, the ring resonators 114 that have been read out are brought back into their storage windows 150a, 150b and the next ring resonators 114 are aligned. Another light pulse is launched, and the acoustic pulse time traces are recorded. This is repeated until the entire row is read out.

It should be noted that if averaging is required for the recorded signals, the ring resonators 114 can remain aligned with the interrogation wavelengths for a necessary averaging time. Once the row readout is completed, the optical switches switch to the next row and the sequence is repeated, until the full array is read out.

The alignment of the ring resonators 114 to the interrogation wavelengths should be done within a timeframe corresponding to a duration between sequential light pulses used to illuminate the sample to generate the photoacoustic ultrasound waves. As an example only, the pulsed laser may allow generating light pulses every 50 ms.

During the alignment process for aligning the resonance wavelength of the ring resonator 114 with the interrogation wavelength, the self-thermo-optic effect needs to be taken into account when this effect is dominant in the behavior of the optical mode.

If not, then the alignment process can be carried out by heating more or less the ring resonator 114. During alignment, the optical interrogation signal may be detected by the photodiodes 164. When a drop in the measured optical intensity is measured on a photodiode 164, the optical mode is coupled to the interrogation wavelength. The sensor 110 can be operated on either the left or the right flank of the resonance transmission curve.

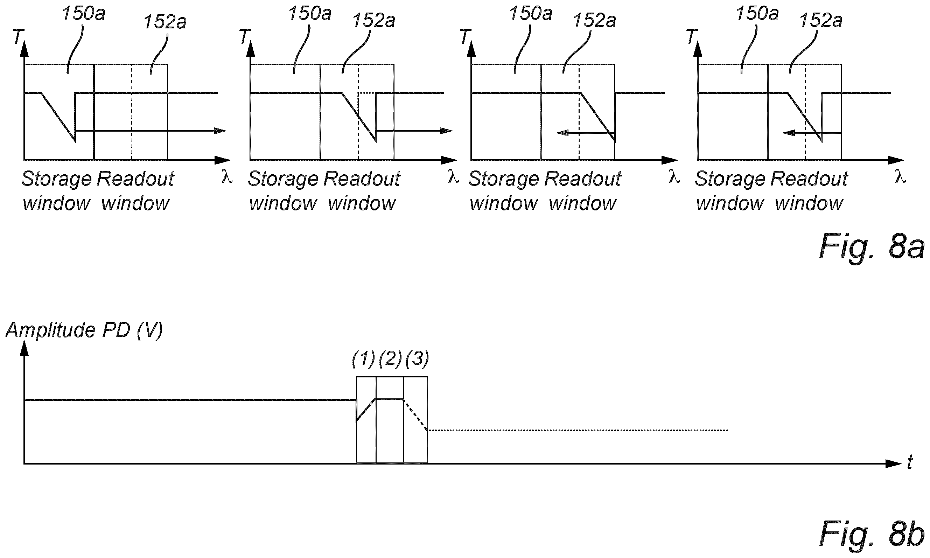

As discussed above, in cases where the self-thermo-optic effect is dominant, only the left flank of the triangular shaped optical mode can be used. In addition, the laser must sweep from the left flank of the mode, in order to find the optimal set point on the resonance transmission curve and avoid hysteresis effects. Hence, in these cases the optical mode may be controlled to be increased (that is, overheated) over the interrogation wavelength so that the optical mode fully passes through the interrogation wavelength, and then allowing the ring to cool down to decrease the resonance wavelength of the optical mode, so that the interrogation wavelength is aligned with the ring resonator 114 by sweeping the optical mode from the left flank. This alignment process is indicated in Fig. 6 for 2 ring resonators 114 aligned and read in parallel. A detailed view of how the optical mode behaves in self-thermo-optic regime during this alignment process is shown in Fig. 8a, indicating in left-most graph the optical mode in the storage window 150a, indicating in the second graph from the left the optical mode passing the interrogation wavelength exhibiting the hysteresis behavior, indicating in the third graph from the left the optical mode as the ring resonator 114 is again cooled down to decrease the resonance wavelength towards the interrogation wavelength and indicating in the rightmost graph the optical mode when aligned with the interrogation wavelength.

The amount of overheating needed for each ring resonator 114 to ensure that the hysteresis behavior is overcome, enabling selecting any set point on the flank of the optical resonance, can be determined in several ways.

A simple way is to heat up every ring resonator 114 with the same thermal power for which it is known that any mode stored in a storage window 150a, 150b will go over the interrogation wavelength arranged in an adjacent readout window 152a, 152b. Assuming that variability in the efficiency of heaters 130 in tuning the resonance wavelength of the ring resonator 130 due to fabrication imperfections is small, this technique could be efficiently implemented.

A second method for controlling the alignment process would be to generate for every sensor device 100 a lookup table storing the needed powers for heating each ring resonator 114 and used every time a ring resonator 114 is to be aligned.

Nevertheless, a more robust alignment process may be needed, especially if the optical properties of the sample vary over time, which is often the case, since optical properties depend on experimental parameters such as temperature. In addition lookup tables, requires a long pre-characterization step of the fabricated sensor devices 100.

A third method for controlling the alignment process that can systematically accomodate fabrication imperfections without the need of lookup tables is now described in relation to Fig. 8b.

In this alignment process, a measured signal on the photodiode may be used. Fig. 8b illustrates the measured signal versus time through the alignment process. The Y-axis of the graph in Fig. 8b represents the voltage output by the photodiode 164, which is proportional to the measured optical intensity. The type of the lines indicates heating (solid line), cooling (dashed line), and no temperature variation of the ring resonator 114 (dotted line, i.e., stationary regime for the heater 130), respectively. The ring resonator 114 is heated up until (1) a sharp decrease in transmitted intensity is observed, marking the fact that the hysteresis behavior has been overcome (see also Fig. 8a, second graph from the left). At this point, the interrogation wavelength is coupled to the optical mode. The ring resonator 114 can be further heated ensure that the full left flank on the optical resonance curve can be exploited, as represented in (1) and (2) of Fig. 8b, or the optimal set point can directly be searched by cooling down the ring. In region (3) of Fig. 8b, the ring resonator 114 is cooled down, enabling searching of the optimal transduction point of the flank of the optical resonance curve. Once it is found, the power in the heater 130 is kept constant to keep the ring resonator 114 at the optimal set point. This completes the alignment process for a ring resonator 114.

The alignment process is based on detecting an abrupt change in the transmission curve, marking the end of the hysteresis behavior of the ring resonator 114. Such an abrupt change cannot be confused with other intensity variations such as Fabry-Perot oscillations occurring in the PIC or variations in the transmisssion efficiencies of grating couplers used for coupling light in and out of a PIC as a function of wavelength. Usually these variations present a smoother evolution as a function of wavelength.

According to an embodiment, a particular method for finding the desired, such as optimal, set point on the flank of the transmission curve may be used.

As discussed above, the optimal alignment point corresponds to the point on the transmission curve which presents maximal slope. A straightforward solution would be to use a lookup table, which would include information of the power that needs to be measured by each photodiode 164 at each desired set point for each ring resonator 114. However, such method requires a long pre-characterization step of the array of sensors 110.

According to an embodiment, the total amplitude of the thermomechanical noise (TMN) may be measured by the photodiode 164 (that is, its integral, given by the variance of a noise time trace dominated by TMN) to know when maximal sensitivity is reached. When the maximal variance is measured, the maximal point of sensitivity has been reached, since the TMN is maximally transduced. Such measurement of TMN does not require a lookup table and is very fast and efficient.

However, using the TMN total amplitude only allows to probe sensitivity for mechanical motions having frequencies around a mechanical resonance frequency of the optomechanical sensor 110. In addition, the optomechanical transduction needs to be sensitive enough to be able to measure the TMN.

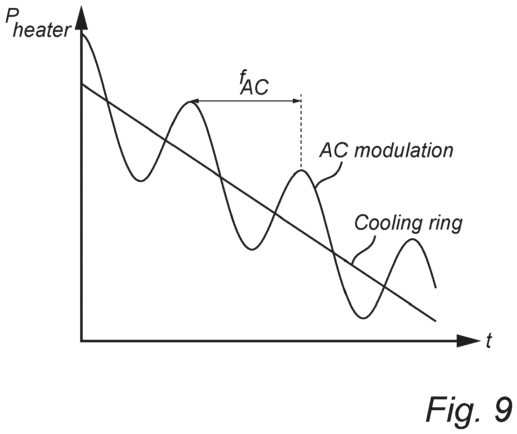

According to an alternative embodiment, the heater 130 associated with the ring resonator 114 may be used for probing in real time the slope of the ring resonator transmission curve. This can be done, while sweeping the left flank of the optical resonance (that is, by cooling the ring resonator), by adding a small AC modulation to the power applied to the heater 130 at a frequency f_{AC}, as illustrated in Fig. 9. This will cause the optical transmission curve to oscillate at the AC frequency in wavelength. Such modulation in wavelength will be transduced on the output from the photodiode 164 as an intensity modulation. By measuring the amplitude of that oscillation, for example via a lock-in detection, the amplitude of the slope can be probed in real time.

This embodiment may be used whether the self-thermo-optic effect deforming the optical mode is present or not. If the transmission curve behavior is dominated by the self-thermo-optic effect (triangular shape), then f_{AC} needs to be comparable to f_{mech typical}, the typical frequencies of the mechanical motion that are to be probed, to make sure that the right slope is probed.

Alternatively, a similar method can be carried out by sweeping the optical mode using the heater 130 and applying a small modulation to the wavelength of the readout laser, if such modulation is allowed by the readout laser.

By using the AC modulation, the slope of the full left flank of the optical mode may be probed, and then by heating up and cooling down the heater 130, the point of maximal sensitivity may be selected. The AC modulation technique requires no lookup table.

The use of the AC modulation presents the advantage of being applicable also in situations where the TMN cannot be measured, for example in cases where the optomechanical transduction is not sensitive enough to resolve it, if systems measuring signals over a wide mechanical band are investigated (i.e., low mechanical quality factors), or if the sensors are operated at low temperatures.

In addition, in the self-thermo-optic-regime, depending on the speed of f_{mech typical} with respect to the thermal time constant of the optomechanical resonator, different slopes of the optical modes, that is, different sensitivities, need to be considered.

Now, optomechanical sensors such as microphones can be operated for measuring mechanical displacements with frequencies f_{mech typical} well below the mechanical resonance frequencies of the optomechanical sensors. In this case, the sensitivity measured with TMN might differ significantly from the one obtained at f_{mech} typical, especially when f_{mech typical} « 1/τ < fₘ₀, where fₘ₀ is the mechanical resonance frequency of the optomechanical sensor. Since it is the sensitivity at f_{mech typical} that is of interest, the use of the AC modulation is advantageous as it can be applied to directly get the information of the desired set point and align the sensor 110 with respect to the interrogation wavelength accordingly.

It should be realized that the AC modulation can be applied also when the self-thermo-optic effect is not dominant. Indeed, an AC modulation can also be applied in a similar way to either the heater 130 or the readout laser 144 to identify the desired set point of the Lorentzian transmission curve.

In the discussion in relation to Fig. 8, storage windows 150a, 150b are illustrated being as wide in wavelength as the readout windows 152a, 152b, for simplicity. However, this is not a necessary condition. Indeed, the size of the readout window 152a, 152b can be reduced in relation to the size of the storage windows 150a, 150b. By doing so, more readout windows 152a, 152b can be introduced in an FSR.

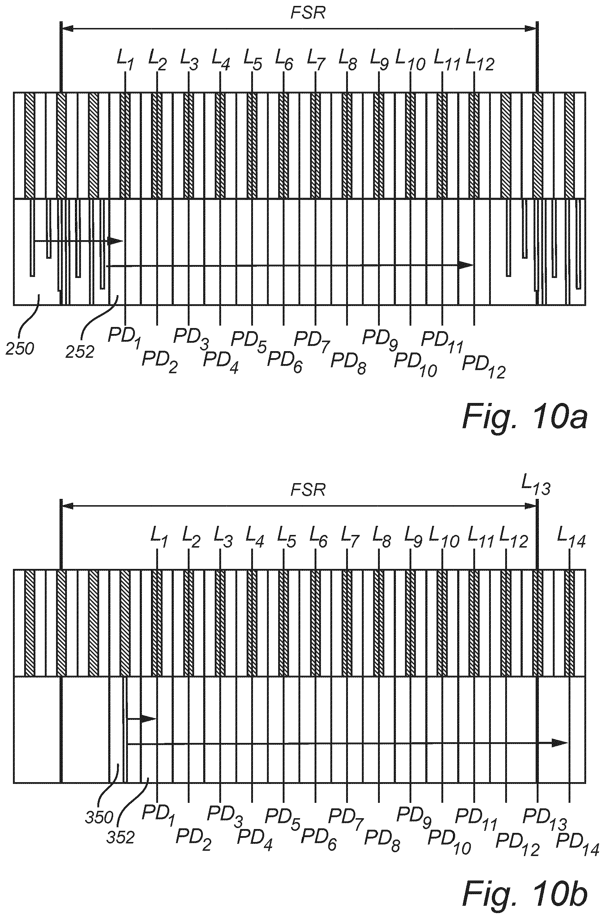

Referring now to Figs 10a-b, embodiments providing configurations wherein the size of the readout windows are not limited by the size of the storage window are illustrated. In Figs. 10a-b, only one storage window 250, 350 is present, with many readout windows 252, 352 of reduced width compared to the width indicated in Fig. 8.

From Figs 10a-b, it is clear that reducing the widths of both the storage window 250, 350 and the readout windows 252, 352 enable to speed up the readout of an entire row of sensors 110, since the narrower each storage window 250, 350 and/or readout window 252, 352 is, the more readout windows 252, 352 can be placed in an FSR. Further, during a single read-out time point, for each readout window 252, 352, the ring resonator 114 of one sensor 110 may be uniquely aligned with the interrogation wavelength of the readout window 252, 352. Hence, having more readout windows 252, 352 implies that more sensors 110 can be read in parallel. The examples shown in Figs 10a-b provide a large number of readout windows 252, 352, and hence provide a high row readout speed.

Fig. 10a illustrates a configuration with a single storage window 250 associated with multiple readout windows 252. In this case, a readout window 252 is 1/3 spectrally wide with respect to a storage window 250, allowing to place a large number (here 12) of readout windows 252 in one FSR. The optical modes that need to be read can all be placed in the same storage window 252. The arrows indicate the heating of an optical mode (here no cooling step for alignment is represented, for simplicity). Vertical lines in the storage window 250 indicate the optical resonance wavelength of different ring resonators 114 (different lengths indicate different optical contrasts). The vertical lines within the readout windows 252 indicate the laser lines providing interrogation wavelengths.

Fig. 10b illustrates another configuration with a single storage window 350 associated with multiple readout windows 352. In the configuration of Fig. 10b, there is a further optimized width of the single storage window 350. The ring resonators 114 are pre-heated by the heater 130 to store the resonance wavelengths before measurements are read out in a narrower wavelength range, for example having a width corresponding to a width allocated for a readout window 352. Since a narrow storage window 350 is used, the spectral space freed in the storage window 350 can now be used to place more readout windows 352 at the other end of the FSR.

By investigating the minimal widths needed for the storage window 250, 350 and readout windows 252, 352, the number of readout windows 252, 352 that may be fitted in an FSR may be maximized. Below, we investigate some methods to determine these parameters.

The storage windows 250, 350 should be sufficiently wide to store all the optical modes of the ring resonators 114 designed with the same nominal diameter but randomly distributed due to fabrication imperfections. A good estimate of the minimal storage window width can hence be determined by carrying out statistics on the spread in the resonance wavelength for the ring resonators 114 for a nominal ring diameter. For example, determining the standard deviation σ of such spread for a given nominal diameter, and then choosing storage windows ranging between the nominal resonance wavelength ±3σ, that is, 6σ wide storage windows 250, would allow storing all the optical modes in the storage windows 250 with great confidence (99.73%) that the optical modes of the ring resonators 114 will be contained in these intervals. By using pre-heating of the ring resonators, the storage windows 350 may be even narrower.

The width of the readout windows 252, 352 should ensure proper readout signals once the optical modes are aligned to the interrogation wavelengths. In Figs 10a-b, for example, there are optical modes aligned simultaneously with adjacent readout windows 252, 352. Hence, if the readout windows 252, 352 do not offer sufficient clearance, cross-talk between signals captured by ring resonators 114 interrogated in adjacent readout windows 252, 352 might occur.

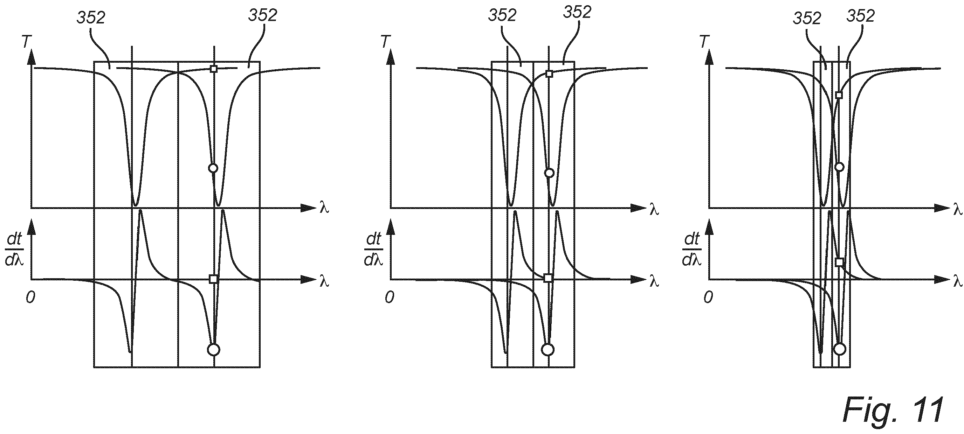

In the case where the shape of the optical modes is not dominated by the self-thermo-optic effects, the narrowest width of the readout window 352 that can be achieved is limited by the width of the Lorentzian optical modes. To give more insight about how to choose the width of the readout windows 352 in these regimes, the amount of cross-talk due to two adjacent optical modes being aligned simultaneously with adjacent interrogation wavelengths is considered, as represented in Fig 11.

Fig. 11 illustrates cross talk analysis between two adjacent readout windows 352 wherein different widths are used for the readout windows. In panels a), b) and c) of Fig. 11, the rectangles indicate the widths of the readout windows 352. The vertical lines indicate the positions of the readout laser lines. Here, a small offset between the readout lasers and the center of the readout windows 352 are present since the optical mode is at the center of the readout window 352, whereas the laser is set on the flank of the optical resonance. The top part of the panels indicates the transmission curves for the two considered ring resonators 114. The bottom part of the panels shows derivatives, that is, the slopes of the transmission curves for the two considered ring resonators 114. Here crosstalk on the ring resonator 114 spectrally located on the right-hand side (at a longer wavelength) of each panel is considered due to the presence of the optical resonance on the left-hand side (at a shorter wavelength). The circles indicate the set point on the transmission curve of the target ring resonator for the readout window at a longer wavelength (top part of the panels), and the corresponding slope at the set point (bottom part of the panels). Laser lines are at the maximum point of sensitivity of the target ring resonator, which correspond to the maximal or minimal value of the slope curve. Here the minimal value of the slope curve is considered. The squares correspond to the points on the transmission curve of the adjacent ring resonator (aligned with the readout window of the shorter wavelength) where the readout laser of the longer wavelength falls. The corresponding slope is indicated by the square in the bottom part of each panel. By reducing the width of the windows (i.e., by going from panel a) to c)), the slope of the curve indicated by the square increases, indicating that the cross-talk increases.

As explained above, a mechanical displacement due to the measurand to be measured, e.g., the passage of an acoustic wave for an OMUS sensor 110, induces a spectral shift of the optical resonance of the ring resonator 114, which is transduced as a variation in optical intensity in the optical interrogation signal and may be measured by measuring the intensity of the transmitted optical interrogation signal. Hence, the sensitivity of such type of optomechanical sensor 110 is directly proportional to the slope of the transmission curve (that is, the derivative of the intensity transmission with respect to wavelength).

It may be assumed that the two ring resonators 114 measure a measurand value, e.g., a mechanical wave, of same amplitude and that the two ring resonators 114 present the same optomechanical coupling strengths. The two previous assumptions are reasonable for an OMUS sensor 110, considering for example that the two sensors 110 read out in parallel are located close on a substrate, meaning that the sensors 110 will measure a mechanical wave of similar amplitude, and that the sensors 110 have been fabricated with similar dimensions, leading to similar optomechanical coupling strengths. In this case, the cross-talk magnitude is equal to the ratio between the transmission slope amplitude of the adjacent ring resonator and the transmission slope amplitude of the target ring resonator, both taken at the wavelength of the readout laser for the target ring resonator (i.e., the amplitudes indicate by the squares and circles in the bottom part of the panels).

From these considerations it is clear that, from panel a) of Fig. 11, where cross-talk is negligible, since the slope amplitude indicated by the square is almost equal to 0, a condition is reached in panel c) of Fig. 11, where the cross-talk becomes significant. Panel b) of Fig. 11 indicates a situation where limited cross-talk is present, while the readout window width is smaller than in the case presented in panel a) of Fig. 11, enabling to stack more readout windows in one FSR.

It should be noted that there may also be other neighbor optical modes that may cause cross-talk in addition to the one considered in Fig. 11 (i.e., the adjacent ring mode at a shorter wavelength). These optical modes are spectrally distributed in the FSR according to a pattern of readout windows 352. Naturally, the cross-talk contributions of all the neighbor ring resonators 114 (aligned with interrogation wavelengths of neighboring readout windows), for example the cross-talk generated by another ring resonator 114 aligned with an interrogation wavelength spectrally located on the right-hand side (at a longer wavelength) of the target ring resonator in Fig. 11, can be summed together to give a total value of the cross-talk. Next nearest neighbor ring resonators 114 can also be summed to the total value of cross-talk if the contribution from such ring resonators 114 is not negligible.

From the discussion presented above, a total cross-talk value can be calculated. The acceptable value of cross-talk may be set according to the sensing performances required for the designed sensor device 100. The minimal width of the readout windows 352 is determined accordingly and is equal to the distance between two adjacent optical modes aligned in their respective readout windows 352.

In order to account for the variability of the optical properties of sensors 110 in an array, the spectrally widest optical mode can be chosen for determining the width of the readout window 352. Alternatively, statistics can be carried out to determine an average optical mode spatial profile for the entire array, and the width of the readout window 352 can be chosen accordingly.

In case the self-thermo-optic effect is dominant, the width of the readout window 352 needs to consider the triangular shape of the optical mode, which is wider than the Lorentzian shape of the mode. In this case, to make sure that the multiple laser lines belonging to adjacent windows are not coupled to the same optical mode, the width of the readout window 352 can be chosen as the width of the triangular shaped optical modes. To account for variability of the optical properties of sensors 110 in the array, the same approaches as when the self-thermo-optic effect is not dominant can be used.

The discussion above in relation to Fig. 11, provide considerations for choosing minimal widths for storage windows 250, 350 and readout windows 252, 352. These considerations can be used for example in the readout schemes presented in relation to Figs 10a-b.

It should be realized, however, that the read-out schemes presented in Figs 10a-b, where only one storage window 250, 350 is present, may be very power consuming if thermal heaters 130 are used for tuning the resonance wavelength. Indeed, some optical modes need to be shifted by almost one FSR, which require large thermo-optic spectral shift. In addition, the high increases of temperature required to shift a ring resonator 114 by one FSR might damage an electrical circuit used for routing electrical signals to the heaters 130, or the heaters 130 themselves.

Alternatively, if capacitive or electro-optical tuning mechanisms are available, such read-out schemes could be implemented in a more realistic way in terms of power budget using a single storage window 250, 350.

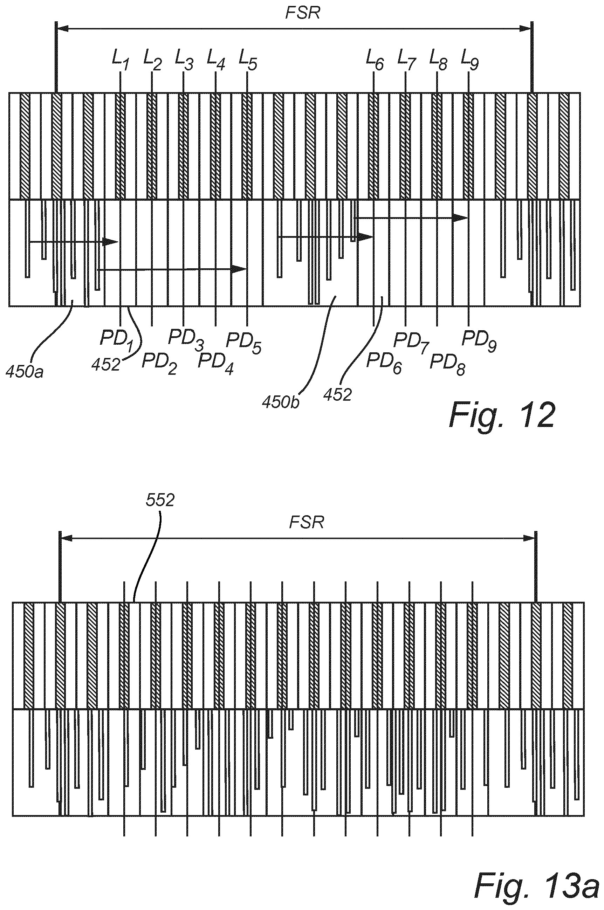

More thermal power economic read-out schemes can also be imagined. For example, alternating readout windows 452 with storage windows 450a, 450b allow to relax the requirement on large spectral shifts, by sacrificing space in the FSR for read-out, such as represented in Fig. 12, and hence row readout speed. In this case as many readout windows 452 as possible are placed next to a storage window 450a, 450b, depending on the efficiency of the heaters 130 in tuning the resonance wavelength of the ring resonators 114 and on the constraints mentioned above.

Another important case to consider is when the spread in resonance wavelength due to fabrication imperfections is so large that only one or less that one 6σ storage window fits in one FSR, as represented in Fig. 13a.

Fig. 13a illustrates a case, where for one nominal ring diameter, the full FSR, or almost the full FSR, is filled by optical modes with the same (if the spread of the resonance wavelengths is smaller than the FSR) or different (if the spread of the resonance wavelengths is larger than the FSR) azimuthal numbers m. In the sensing of the measurand, it may be possible to use optical modes with different azimuthal numbers.

It should be noted that in the case illustrated in Fig. 13a, the optical modes cannot be directly aligned to the readout laser lines. Indeed, another optical mode could also be aligned by accident to the readout laser lines, due to the random distribution of the resonances. Some clearance space around the readout lasers needs to be ensured to avoid the alignment (and hence cross-talk) of multiple rings to the same laser line. This minimal amount of clearance is given by the minimal width of a readout window 552 which can be determined as discussed above, and as indicated in Fig 13a.

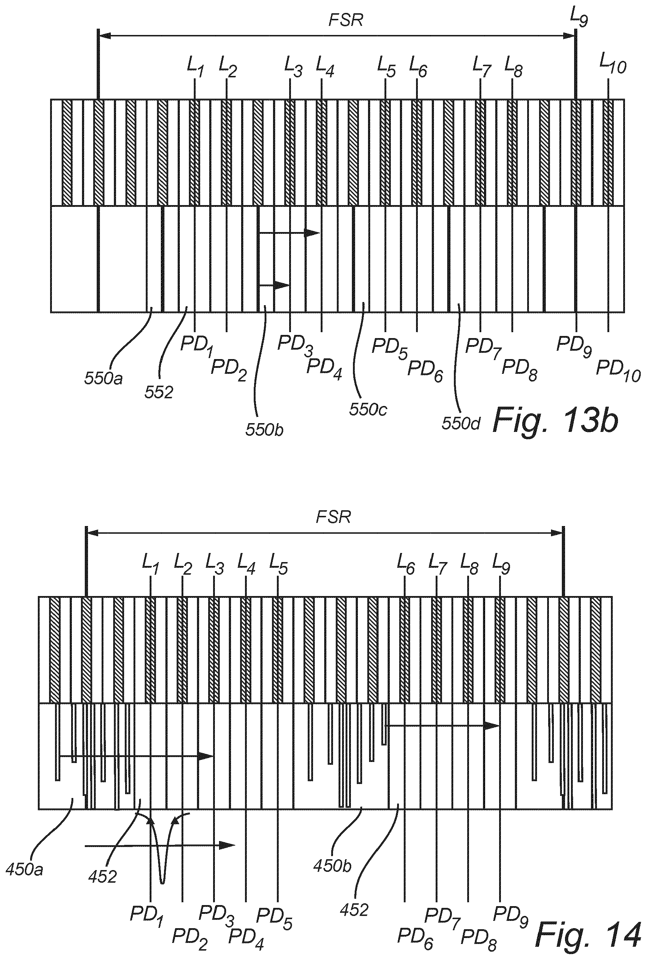

In order to avoid accidental cross-talk, in this case, the optical modes can be subdivided into new storage windows 550a-d, as shown in Fig. 13b. The ring resonators 114 having resonance wavelengths within wavelength ranges associated with readout windows 552 are then pre-heated to the nearest storage window 550a-d associated with a longer wavelength, so that the corresponding optical modes of these ring resonators 114 all spectrally overlap into a pre-heated storage window 550a-d, as represented in Fig. 13b.

After such pre-heating, the space now available in the FSR can be used to define readout windows 552, in which laser lines can be coupled. As indicated by arrows in Fig. 13b, optical modes are pulled out, aligned to the readout laser lines for read-out, and may then be brought back into the pre-heated storage windows 550a-d.

The fewer and the narrower the pre-heated storage windows 550a-d need to be used, the more readout windows 552 can be defined, and the faster the read-out scheme will be.

Since the pre-heated storage windows 550a-d are surrounded by readout windows 552, the width of the optical modes of sensors 110 arranged in the storage windows 550a-d should be set to prevent cross-talk between the stored optical modes with the adjacent optical modes aligned for read-out. Hence, the minimal width of the storage windows 550a-d corresponds to the minimal width of a readout window 552, as indicated in Fig. 13b.

The spectral width of multiple readout windows 552 between neighboring storage windows 550a-d, i.e., the spectral spread between storage windows 550a-d, will define the number of readout windows 552 that are available. The fewer and the wider these intervals between neighboring storage windows 550a-d may be, the more space within an FSR will be available to define readout windows 552, resulting in faster measurements. The maximal width of the intervals is limited by the efficiency of the heaters 130 and the maximal temperature and power constraints that the array of sensors 110 can withstand.

The pre-alignment procedure for arranging the ring resonators 114 in the pre-heated storage windows 550a-d can be carried out during or before using the sensing device 100 for its sensing application. In the case of OMUS sensors 110, the ring resonators 114 would be distributed as represented as in Fig. 13b during or before the photoacoustic imaging experiment is carried out.

To this end, transmission measurements can be carried out by the detector 160 to make sure that the configuration of Fig. 13b is reached. This can be checked by providing a signal in the waveguide 120 from a tunable laser, sweeping the tunable laser over the FSR and measuring the transmitted intensity with a photodiode. Alternatively, light generated from a broadband light source can be sent into the waveguide 120, and a dispersive detector such as spectrometer or an optical spectrum analyzer can be used to obtain the desired spectrum.

In addition, the alignment procedure presented in Fig. 13b is relevant in situations where the array of sensors 110 has been designed with 6σ storage windows smaller than the FSR, but where the readout windows intervals associated with one storage window 550a-d are used as the storage window for the next group of ring resonators 114 of a different diameter. This case can be obtained by placing storage windows 550a-d (i.e., by controlling the ring diameters) in such a way that the storage windows 550a-d associated with different ring diameters are directly adjacent, without leaving any spectral clearance for readout windows. Then, the ring resonators 114 may be pre-heated to ensure that the ring resonators 114 are brought to pre-heated storage windows 550a-d providing spectral space for readout windows 552.

Finally, although the alignment scheme discussed above in relation to Fig. 13b seems to be particularly efficient as it allows to read out ring resonators 114 with a strong dispersion in the resonance wavelengths due to fabrication imperfections, the implementation requires to control the resonance wavelength of all the ring resonators 114 on the same row during the pre-heating step, which can be challenging for large scale arrays of optomechanical sensors 110 and a power consumption of the heaters 130 will be high. Hence, the previously discussed read-out schemes that do not require any pre-heating are also interesting.

Between all the options discussed above, the chosen distribution of readout windows and storage windows within one FSR depends on the spread in resonance wavelength of the designed sensors, the efficiency of the heater, the required read-out speed, the temperature and the power budget constraints, and so on.

Referring now to Fig. 14, a first approach for aligning the resonance wavelengths of a plurality of ring resonators 114 to respective selected interrogation wavelengths will be discussed.

According to the first approach, during the alignment of the optical modes from the storage windows 450a, 450b to the readout windows 452, each ring resonator 114 is aligned sequentially (that is, one after the other). In this case, wavelength demultiplexing can be carried out in a direct way, as by knowing which heater 130 is heated up, that is, in which bond pad connected to the heaters 130 current is provided, it is known which ring resonator 114 that is being aligned. As mentioned before, the optical mode is aligned to the targeted laser line (selected interrogation wavelength) when a drop in optical intensity is measured on the corresponding photodiode 164.

It should be noted that during the heating up of the ring resonator 114, the optical interrogation signal including light from the readout lasers can be used for sampling the spectral position of the optical mode, as indicated in Fig. 14. As shown in bottom part of Fig. 14, the optical transmission measured by two photodiodes (PD1 and PD2) based on a spectral position of the optical mode is indicated by triangles. Measuring evolution of the transmitted intensities during heating of the ring resonator 114 allows monitoring where the optical mode is spectrally located during the heating and cooling process. This provides knowledge of where the heated mode is located during the alignment process.

In addition, during the alignment process, the self-thermo-optic effect needs to be considered, if this effect dominates the behavior of the optical modes. Indeed, in case of the self-thermo-optic effect, the power coupled to the optical mode is determinant of the optical behavior of the optical mode. As shown in Fig 2, the transmission is triangular when high power is coupled to the optical mode, and the latter can present a hysteresis behavior.

When light is coupled to the optical mode, any decrease in the optical intensity that is coupled to the mode cools down the optical mode, and hence can decouple the laser light.

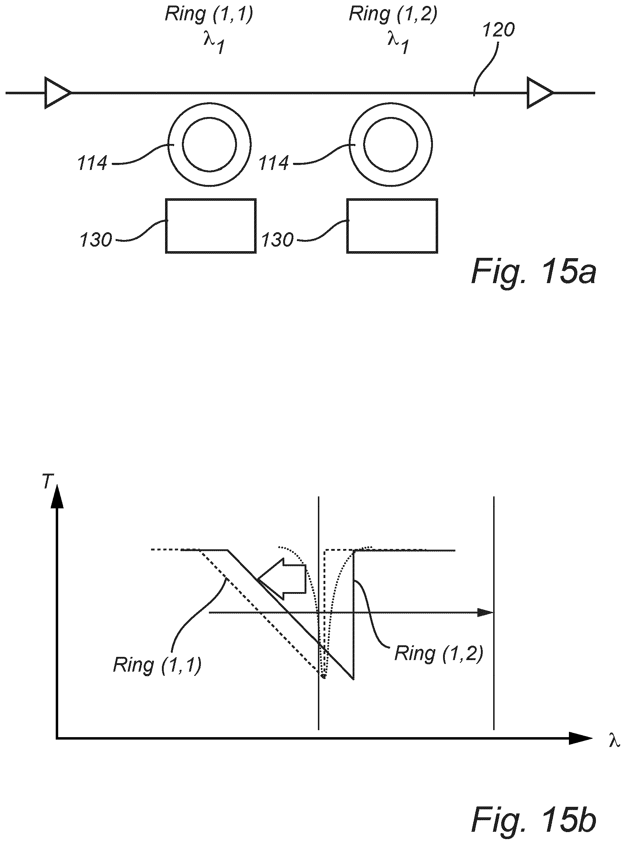

Fig. 15a illustrates two ring resonators 114, named Ring (1,1) and Ring (1,2), belonging to the same storage window. The triangles indicate the light direction of propagation in the waveguide 120.

Fig. 15b illustrates the optical modes of Ring (1,1) and Ring (1,2) during the alignment process. The optical mode indicated by a solid line is the optical mode associated with Ring (1,2) and is illustrated as being already coupled to a readout laser line (vertical line at a short wavelength). The position of the Lorentzian mode corresponding to Ring (1,2) is also shown as indicated by a dotted line.

The optical mode indicated by a dashed line represents the optical mode associated with Ring (1,1) which is being aligned with another readout laser line (vertical line at a long wavelength), as represented by the thin arrow. In the current configuration, as Ring (1,1) is arranged spatially before Ring (1,2), i.e., the signal in the waveguide 120 passes Ring (1,1) before passing Ring (1,2) (see Fig. 15a), upon the passage of the optical mode towards a longer wavelength, Ring (1,1) couples the light that was coupled to Ring (1,2). The light intensity coupled to Ring (1,2) decreases, meaning that Ring (1,2) cools down (indicated by the thick arrow in Fig. 15b). After the optical mode associated with Ring (1,1) has finished crossing the wavelength of the optical mode of Ring (1,2), Ring (1,2) is not coupled to its readout line anymore. Swapping the order of alignment solves the problem, as in such case the optical mode that is being aligned couples to the light in the waveguide 120 which has already been coupled to the other ring.

Hence, when the optical modes are dominated by the self-thermo-optic effect, special care should be taken in the order of aligning the optical modes.

To avoid this issue, crossing between optical modes should be avoided. This can be carried out by making sure that optical modes of ring resonators 114 are first aligned with the readout windows located farthest away from the storage windows.

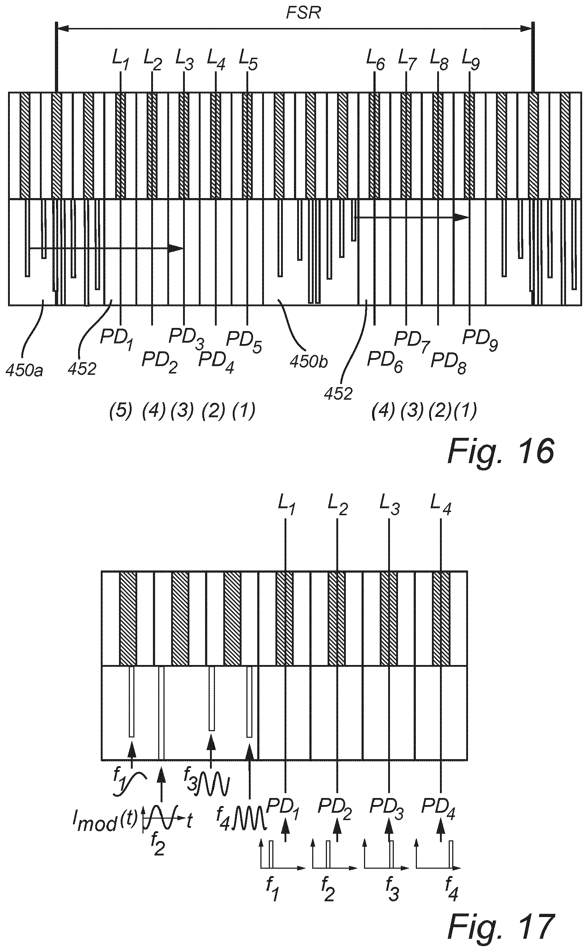

Fig. 16 illustrates a sequence of alignment time steps that may be used for a storage windows 450a, 450b and readout windows 452. Hence, the optical mode being aligned first for a storage window is aligned to the laser line indicated by (1), followed by an optical mode being aligned to the laser line indicated by (2), and so on.

In case crossing between optical modes are needed during the alignment process, the optical ring resonator 114 associated with the mode that is being aligned should be spatially located in relation to the waveguide 120 after the ring resonators 114 that are already aligned to their laser lines. In other words, the ring resonator 114 being aligned should be located farther away from the laser light source with respect to the ring resonators 114 already aligned.

If all ring resonators 114 that are to be simultaneously read out are aligned in parallel, the alignment process may be speeded up. In this case, all the heaters 130 of sensors 110 that are selected to be aligned are turned on simultaneously. This solution is attractive, but also poses challenges. Indeed, there is a need to know the correspondence between the heated-up heaters 130, that is, the used bond pads, and the optical modes which are being aligned.

One solution is to have prior knowledge about the sensor device 100, knowing, through a pre-characterization step, which optical mode stored in the storage window belongs to which ring resonator 114.

Alternatively, modulation of the resonance wavelength of each optical mode is also an effective approach. As illustrated in Fig. 17, the resonance wavelength of each ring resonator 114 may thus be modulated at a different modulation frequency by applying an AC current to the heaters 130. The spectra of the intensity signals measured by the photodiodes 164 are measured, and by knowing which tones (that is, which frequencies) are measured at the outputs of the photodiodes 164, it is known which ring resonator 114 is aligned to the photodiode 164 and hence the corresponding laser line. This also allows verifying if multiple ring resonators 114 are coupled to the same laser line, enabling such situations to be avoided, which are unwanted for the presented multiplexing schemes.

As illustrated in Fig. 17, four different frequencies f₁, f₂, f₃, f₄ may be used for providing modulations of the AC current to the heaters 130. The signals detected by the photodiodes PD₁, PD₂, PD₃, PD₄ each measures a unique modulated intensity frequency, allowing unambiguous identification of which ring resonator is aligned with which laser line.

It should be noted that if the behavior of the optical modes is dominated by the self-thermo-optic effect, this needs to be taken into account in terms of mode crossing and decoupling of optical modes from laser lines, as explained before.

Also, it should be noted that the modulation of the resonance wavelength may also be used for finding the desired set point on the flank of the optical resonance curve, as explained above in relation to Fig. 9.

In the read-out schemes presented above, multiple ring resonators 114 belonging to the same storage window and ring resonators 114 belonging to different storage windows will be heated in parallel. For example, in the case of Fig. 11, 9 ring resonators 114 can be read in parallel. This implies that at each read-out time point, 9 heaters 130 associated with respective ring resonators 114 are running in parallel in the sensor device 100.

While reading out multiple ring resonators 114 in parallel, the thermal cross-talk between the ring resonators 114 may need to be avoided or minimized.

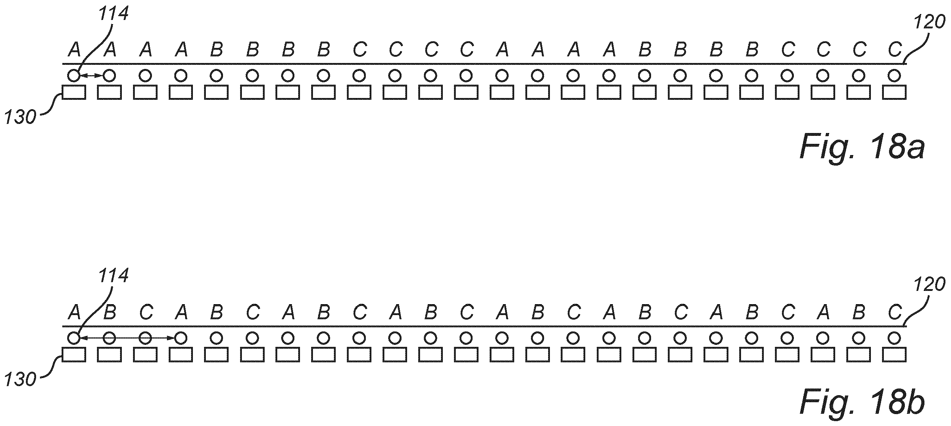

The thermal cross-talk is discussed in relation to Fig. 18a. In the embodiment illustrated in Fig. 18a, 2 storage windows storing 12 ring resonators 114 each are provided. It is further supposed that there is spectral space in one FSR to place 4 readout windows per storage window. This means that 8 ring resonators 114 may be read out in parallel (i.e., 4 per window), and the total of 24 ring resonators 114 are read in 3 time steps of 3 different read-out time points.

The ring resonators indicated by "A" are read out in the first time step, the ring resonators indicated by "B" are read out in the second time step, and the ring resonators indicated by "C" are read out in the third time step.

It is further supposed that during the first time step (time step A) the third ring resonator from the left-hand side of Fig. 18a has been already aligned to a readout laser line, and that the fourth ring resonator is about to be aligned. By increasing the thermal power in the heater associated with the fourth ring resonator, the heat generated by the turned-on heater might misalign the third ring resonator. Compensating for these effects on the third ring resonator might lead to complicated alignment processes if the ring resonators 114 are affected by the heat of their neighbors.

Hence, the set of selected sensors 110 to be read out in parallel may be selected more wisely. In the read-out scheme indicated in Fig. 18a, strong thermal cross-talk effects may be provided, since the cross-talk distance (that is, the smallest distance between two ring resonators 114 read out in parallel) is equal to the pitch in the array. Therefore, another read-out scheme may be desirably used and/or thermal cross-talk may be avoided in other manners. However, it should be realized that, in some embodiment, the read-out scheme illustrated in Fig. 18a may be used. For instance, if a risk of thermal cross-talk is low.

One solution is to decrease the thermal cross-talk effects by increasing the distance between the ring resonators 114 that are read out in parallel. To this end, one efficient approach is to take advantage of the fact that not all the ring resonators 114 in a storage window can be read in parallel. This can be due to the limited number of readout windows, or the number of laser lines and/or photodiodes available.

When this is the case, an interesting strategy is to select ring resonators 114 that are arranged next to each other to be read out at different time steps. In this way, thermal cross-talk affects mainly ring resonators 114 that will be read out at different time steps, leading to reduced impact of the thermal cross-talk on the overall process. In other words, the cross-talk distance is increased to a scale of p ×pitch in the array, where p is the number of time steps needed to read a full row.

This is illustrated in Fig. 18b, for p = 3 as discussed before. It should be noted that using this approach ensures that the row is read out with the same number of time steps, that is, with the same speed, as in the case illustrated in Fig. 18a, where adjacent ring resonators 114 are read out in parallel.

In practice, ring resonators 114 belonging to different storage windows will have very similar diameters. To shift the resonance wavelength of a ring resonator by few nanometers, a difference in radius of few tens of nm between the ring resonators needs to be introduced. This implies that the cross-talk distance in Fig. 18b is practically always the same for every ring resonator read out in parallel, even if the ring resonators belong to different storage windows. This simplifies the modelling of large arrays of thermally tuned sensors, since only one cross-talk distance needs to be considered.

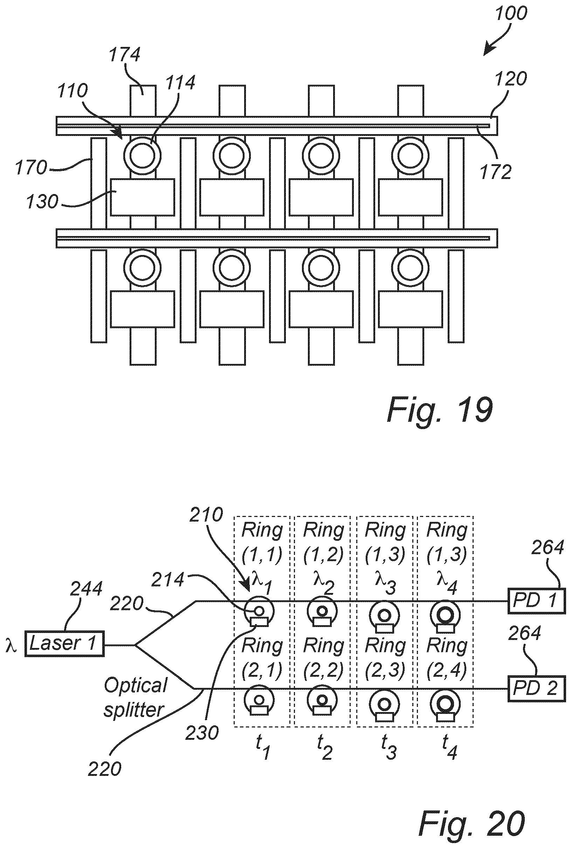

Referring now to Fig. 19, it should be noted that the effect of thermal cross-talk could also be reduced by introducing trenches 170 between OMUS sensors 110 arranged on a common substrate, such as a semiconductor die. The trenches 170 can be filled with air, vacuum, silicon oxide, or any other material presenting poor thermal conductivity with respect to silicon. This would reduce the heat flux going from one ring resonator 114 to its neighbors.

By leaving the trenches 170 open, that is, no material being arranged in the trenches 170, but just air or vacuum, or by having water or a poorly acoustic conductive material in the trenches 170, the trenches 170 can also prevent or reduce acoustic cross-talk between the sensors 110, that is, the motion of a membrane 112 is not affected by its neighbors.

For an array where the ring resonators 114 are read row by row, trenches 170 and/or thermal isolations may be needed only between the columns of the rings, as different rows are read at different time instants. Further, by not having any trenches along rows, the sensor device 100 is provided with more space for the fabrication of the heaters 130 and/or other optical and/or electrical components that could be useful for read-out from the array of sensors 110.

In addition, the trenches 170 may need to be arranged to let the waveguides 120 pass through the whole array column-wise (i.e., along an entire row). Such openings in the trenches 170 can be used to also to route metal lines 172 below the optical waveguides 120, whereas along the direction of columns, another level of metal lines 174 can be routed easily, since no trenches are present between rows. Hence, the layout shown in Fig. 19 allows routing electrical signals in both the vertical and horizontal direction (along rows and columns), enabling designing of complex 2D electrical circuits to bring desired currents to the targeted heaters 130. This can be done while thermally and acoustically isolating the ring resonators 114 to limit cross-talk and having space between the ring resonators 114 in the vertical direction to add extra optical and/or electrical components.

As discussed above in relation to Figs 7a-b, the read-out of the array of sensors 110 may be based on methods to multiplex all signals in a row into a single waveguide 120. Additionally, methods to systematically read out many sensors 110 through a single waveguide 120 have been discussed. These methods are based on the dynamical tuning of the ring resonators 114, for example by using heaters 130. The free spectral range is divided into storage windows and readout windows. To readout the full array of sensors 110, in the embodiments described above, the rows are read sequentially, that is, the time multiplexing degree of freedom is implemented row-wise. It should be noted, however, that this is not a necessary condition. The current architecture would also allow time multiplexing to be implemented column-wise.

An embodiment implementing time multiplexing column-wise is shown in Fig. 20. In this embodiment, the laser 244 is tuned to the ring resonators 214. For instance, a tunable laser 244 is used. It should be noted that the column-wise time multiplexing may instead be implemented with a laser having a fixed wavelength and tunable ring resonators, by using the notions of storage windows and readout windows, allowing to implement time multiplexing column-wise. For example, all the ring resonators of a row (and hence in the full array) may have identical diameters and may be stored in the same storage window distributed. All the ring resonators belonging to the same column may be pulled out simultaneously and aligned in the readout window where the readout laser line is located.

The light from the laser 244 is split by an optical splitter so as to be provided into plural waveguides 220 at the same time. The laser wavelength is coupled simultaneously to all the ring resonators 214 in one column, i.e., to ring resonators 214 in each row.

One photodiode 264 is needed per row and only one laser 244 is needed, reducing the number of necessary light sources. The necessary power of the readout laser increases with the number of sensors 210 simultaneously readout.

Each of the photodiodes 264 may thus detect measurements from one sensor 210 at a time in the row with which the photodiode 264 is associated. The wavelength of the laser 244 may be swept to align the resonance wavelengths of different ring resonators 214 located in different columns spectrally to the laser wavelength. The heaters 230 may be used for precisely setting the resonance wavelengths of the ring resonators 214 at the same value within the same column, so that all ring resonators 214 are aligned with the laser wavelength.

From these two approaches of row-wise time multiplexing and column-wise time-multiplexing, combinations of optical splitters and switches may be used, that is, combinations of row and column readout (i.e., time, wavelength and space multiplexing), can be proposed to limit the number of necessary readout lasers and the power required per readout laser.

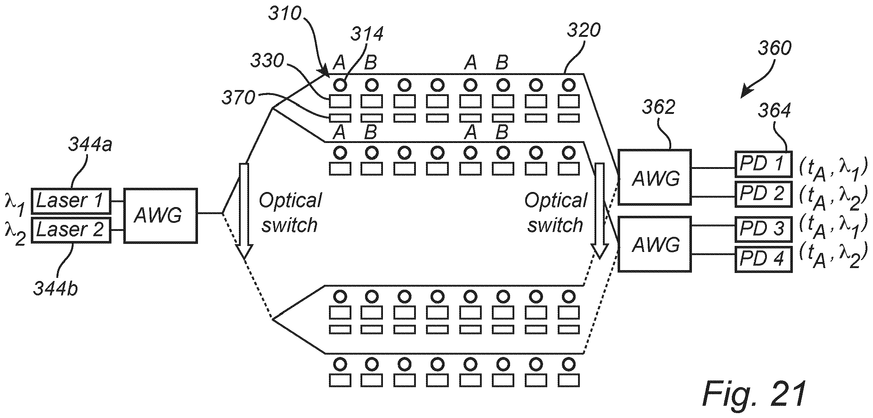

An embodiment is illustrated in Fig. 21, where multiple bundled rows are simultaneously interrogated via an optical power splitter such that an optical interrogation signal is provided to a plurality of waveguides 320, each associated with a unique row in the bundled rows. Once the bundled rows are read out, optical switches switch to the next bundled rows, and the process is repeated.

The rows are separated by isolation trenches 370. Ring resonators 314 are shown in different sizes with exaggerated difference in size for illustration purposes. Ring resonators 314 of different sizes have different nominal resonance wavelengths. Heaters 330 are used for aligning the ring resonators 314 to the fixed wavelengths of lasers 344a, 344b according to the storage and readout windows principles. A, B symbols show different readout time intervals. The simultaneously read out sensors 310 are scattered over the array row-wise, to prevent thermal cross-talk.

In this embodiment, to simultaneously read out 4 sensors 310, only 2 lasers 344a, 344b are necessary. The waveguides 320 provide optical interrogation signals to a detector 360 which comprises separate detector units receiving the signals from separate waveguides 320. Each detector unit may comprise an AWG component 362 and a bank of photodiodes 364. The detector unit may split the optical interrogation signal from the waveguide 320 connected to the detector unit into different signal components, each having a unique interrogation wavelength, and the signal components may be detected by the bank of photodiodes 364.

Once the bundled rows are fully read out, the optical switches switch to the next bundle of rows for providing the optical interrogation signal from the lasers 344a, 344b to the waveguides 320 associated with the new bundle of rows and for providing the optical interrogation signals propagated by the waveguides 320 associated with the new bundle of rows to the detector 360. Then, the measurement process is repeated.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A sensor device (100) comprising:
a plurality of sensors (110; 210; 310), wherein each sensor (110; 210; 310) comprises an optical resonance element (114; 214; 314), wherein an optical characteristic of the optical resonance element (114; 214; 314) is configured to be affected by a measurand to be measured by the sensor (110; 210; 310);
a waveguide (120; 220; 320) associated with a group of sensors among the plurality of sensors, wherein the waveguide (120; 220; 320) is configured to receive an optical interrogation signal comprising at least one interrogation wavelength;
a plurality of control elements (130; 230; 330), wherein each sensor (110; 210; 310) in the plurality of sensors is associated with a unique, individual control element (130; 230; 330) of the plurality of control elements, wherein each control element (130; 230; 330) is individually controllable and configured to control a resonance wavelength of optical resonance of the sensor (110; 210; 310) associated with the control element (130; 230; 330) for tuning the resonance wavelength of the sensor (110; 210; 310);
wherein the sensor device (100) is configured for the resonance wavelength of each of the sensors (110; 210; 310) in the group of sensors, before read-out of measurements from the group of sensors, being in at least one wavelength range (150a, 150b; 250; 350; 450a, 450b; 550a-d), wherein the at least one interrogation wavelength is outside the at least one wavelength range (150a, 150b; 250; 350; 450a, 450b; 550a-d);
wherein the sensor device (100) is configured to, for read-out of measurement from a selected sensor (110; 210; 310) among the group of sensors, tune the resonance wavelength of the selected sensor (110; 210; 310) by controlling the control element (130; 230; 330) to which the selected sensor (110; 210; 310) is associated for aligning the resonance wavelength with a selected interrogation wavelength of the at least one interrogation wavelength;
wherein the group of sensors are configured to be controlled for arranging, at a single read-out time point, the selected sensor (110; 210; 310) to be uniquely aligned with the selected interrogation wavelength for reading out the measurand measured by the selected sensor (110; 210; 310) using the selected interrogation wavelength.

2. The sensor device according to claim 1, wherein each of the control elements comprises a heater (130; 230; 330) for heating the optical resonance element (114; 214; 314) of the sensor (110; 210; 310) associated with the control element, wherein the heater (130; 230; 330) is configured to be controlled, for aligning the resonance wavelength with the selected interrogation wavelength, to heat the optical resonance element (114; 214; 314) to change the resonance wavelength from the at least one wavelength range (150a, 150b; 250; 350; 450a, 450b; 550a-d) to a wavelength larger than the selected interrogation wavelength and thereafter cool the optical resonance element (114; 214; 314) to bring the resonance wavelength to match the selected interrogation wavelength.

3. The sensor device according to claim 2, further comprising a detector (160; 264; 360) configured to detect the at least one interrogation wavelength, wherein the detector (160; 264; 360) is configured to detect intensity of the at least one interrogation wavelength during alignment of the resonance wavelength of the selected sensor (110; 210; 310) with the selected interrogation wavelength for identifying a set point for alignment.

4. The sensor device according to claim 2 or 3, wherein the sensor device (100) is configured for the resonance wavelength of each of the sensors (110; 210; 310) in the group of sensors, before read-out of measurements from the group of sensors, being in the at least one wavelength range (350; 550a-d), by the sensor device (100) being configured to provide control signals to the heater (130; 230; 330) of each of the control elements for controlling the resonance wavelength of the sensor (110; 210; 310) associated with the control element (130; 230; 330) to be tuned to the at least one wavelength range (350; 550a-d).

5. The sensor device according to any one of claims 1-3, wherein the sensor device (100) is configured for the resonance wavelength of each of the sensors (110; 210; 310) in the group of sensors, before read-out of measurements from the group of sensors, being in the at least one wavelength range (150a, 150b; 250; 450a, 450b), by the sensor device (100) being manufactured with tolerances ensuring that a nominal resonance wavelength of each sensor (110; 210; 310) is within the at least one wavelength range (150a, 150b; 250; 450a, 450b).

6. The sensor device according to any one of the preceding claims, wherein the sensor device (100) is configured to, during alignment of the resonance wavelength of the selected sensor (110; 210; 310) with the selected interrogation wavelength, modulate a control signal to the control element (130; 230; 330) to which the selected sensor (110; 210; 310) is associated or modulate the interrogation wavelength, using a modulation frequency.

7. The sensor device according to any one of the preceding claims, wherein the optical interrogation signal comprises a plurality of separate interrogation wavelengths, wherein the sensor device (100) is configured for simultaneous read-out of measurements from a set of selected sensors (110; 310) among the group of sensors, wherein, for each of the selected sensors (110; 310), the sensor device (100) is configured to tune the resonance wavelength to match a unique interrogation wavelength of the plurality of separate interrogation wavelengths.

8. The sensor device according to claim 7, wherein the sensor device (100) is configured for arranging the resonance wavelength of each of the sensors (110; 310), before read-out of measurements from the sensors (110; 310), in a plurality of distinct wavelength ranges (150a, 150b; 450a, 450b; 550a-d), wherein the plurality of separate interrogation wavelengths comprises a plurality of sets of multiple interrogation wavelengths, each set of interrogation wavelengths being associated with a unique wavelength range (150a, 150b; 450a, 450b; 550a-d).

9. The sensor device according to claim 7 or 8, wherein the sensor device (100) is configured for controlling read-out of measurements from a plurality of sets of selected sensors (110; 310) among the group of sensors, wherein the sensor device (100) is configured to time-multiplex read-out of measurements from different sets of selected sensors (110; 310).

10. The sensor device according to claim 9, wherein the sensor device (100) is configured to control selected sensors (110; 310) within different sets to be physically arranged interlaced with each other.

11. The sensor device according to any one of claims 7-10, wherein the plurality of separate interrogation wavelengths is within a free spectral range of optical resonance elements (114; 314) of the plurality of sensors (110; 310).

12. The sensor device according to any one of the preceding claims, wherein the sensor device (100) is configured for controlling read-out of measurements from a plurality of groups of sensors, wherein the sensor device (100) is configured to time-multiplex read-out of measurements from different groups of sensors.

13. The sensor device according to claim 1, wherein the waveguide (220; 320) forms part of a plurality of waveguides, wherein each waveguide (220; 320) is associated with a unique group of sensors, wherein a group of waveguides (220; 320) among the plurality of waveguides are configured to simultaneously receive the optical interrogation signal for simultaneous read-out of measurement from selected sensors (210; 310) from the groups of sensors associated with the group of waveguides (220; 320).

14. A photo-acoustic imaging device comprising:
the sensor device (100) according to any one of the preceding claims, wherein each sensor (110; 210; 310) of the plurality of sensors comprises an opto-mechanical element (112) for receiving an acoustic wave forming the measurand, and wherein the opto-mechanical element (112) is configured to be affected by the received acoustic wave so as to change the optical characteristic of the optical resonance element (114; 214; 314).

15. A method for read-out of a detected measurand, said method comprising:
receiving an optical interrogation signal comprising at least one interrogation wavelength in a waveguide associated with a group of sensors among a plurality of sensors, wherein each sensor comprises an optical resonance element, wherein an optical characteristic of the optical resonance element is configured to be affected by the measurand to be measured by the sensor, wherein, before read-out of measurements from the group of sensors, the resonance wavelengths of each of the sensors in the group of sensors is in at least one wavelength range, wherein the at least one interrogation wavelength is outside the at least one wavelength range; and
tuning the resonance wavelength of a selected sensor by controlling a control element to which the selected sensor is associated for aligning the resonance wavelength with a selected interrogation wavelength of the at least one interrogation wavelength; and
reading out the detected measurand from the selected sensor by the optical interrogation signal, wherein the selected sensor is during read-out uniquely aligned with the selected interrogation wavelength for reading out the measurand.
